(19) Europäisches Patentamt
European Patent Office
Office européen des brevets

(11) EP 2 947 091 B1

(12) EUROPÄISCHE PATENTSCHRIFT

(45) Veröffentlichungstag und Bekanntmachung des Hinweises auf die Patenterteilung:
**05.10.2016 Patentblatt 2016/40**

(51) Int Cl.:
***C07F 9/6574*** (2006.01) ***B01J 31/18*** (2006.01)
***C07C 45/50*** (2006.01) ***C07F 9/145*** (2006.01)

(21) Anmeldenummer: **15166039.6**

(22) Anmeldetag: **30.04.2015**

# (54) GEMISCHE VON MONOPHOSPHITLIGAND UND DEREN VERWENDUNG ZUR KATALYSE EINER HYDROFORMYLIERUNGSREAKTION

MIXTURES OF MONOPHOSPHITE LIGAND AND THEIR USE IN THE CATALYSIS OF A HYDROFORMYLATION REACTION

MÉLANGES DE LIGANDS DE MONO-PHOSPHITE ET LEUR UTILISATION POUR LA CATALYSE D'UNE RÉACTION DE HYDROFORMYLATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2014 DE 102014209533**

(43) Veröffentlichungstag der Anmeldung:
**25.11.2015 Patentblatt 2015/48**

(73) Patentinhaber: **Evonik Degussa GmbH**
**45128 Essen (DE)**

(72) Erfinder:
- **Dyballa, Katrin Marie**
  **45657 Recklinghausen (DE)**
- **Franke, Robert**
  **45772 Marl (DE)**
- **Geilen, Frank**
  **45721 Haltern am See (DE)**
- **Hess, Dieter**
  **45770 Marl (DE)**
- **Fridag, Dirk**
  **45721 Haltern am See (DE)**

(56) Entgegenhaltungen:
**EP-A2- 0 614 870** **WO-A1-2010/057099**
**DE-A1-102011 085 883**

- **RUI M B CARRILHO ET AL: "Rhodium/tris-binaphthyl chiral monophosphite complexes: Efficient catalysts for the hydroformylation of disubstituted aryl olefins", JOURNAL OF ORGANOMETALLIC CHEMISTRY, ELSEVIER-SEQUOIA S.A. LAUSANNE, CH, Bd. 698, 5. Oktober 2011 (2011-10-05), Seiten 28-34, XP028392107, ISSN: 0022-328X, DOI: 10.1016/J.JORGANCHEM.2011.10.007 [gefunden am 2011-10-29]**
- **KONSTANTIN N GAVRILOV ET AL: "Phosphites and diamidophosphites based on mono-ethers of BINOL: a comparison of enantioselectivity in asymmetric catalytic reactions", TETRAHEDRON, ELSEVIER SCIENCE PUBLISHERS, AMSTERDAM, NL, Bd. 68, Nr. 5, 28. November 2011 (2011-11-28), Seiten 1581-1589, XP028436933, ISSN: 0040-4020, DOI: 10.1016/J.TET.2011.11.092 [gefunden am 2011-12-06]**
- **ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803**
- **ANA Z. GONZÁLEZ ET AL: "Gold(I)-Catalyzed Enantioselective [4 + 2]-Cycloaddition of Allene-dienes", ORGANIC LETTERS, Bd. 12, Nr. 1, 1. Januar 2010 (2010-01-01) , Seiten 200-203, XP055201269, ISSN: 1523-7060, DOI: 10.1021/ol902622b**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Gemische von Monophosphitligand und deren Verwendung zur Katalyse einer Hydroformylierungsreaktion.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor $P^{III}$. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Die Art des Katalysatorsystems und die optimalen Reaktionsbedingungen für die Hydroformylierung sind von der Reaktivität des eingesetzten Olefins abhängig.
Die unterschiedliche Reaktivität von isomeren Octenen ist ebenfalls bekannt (siehe B. L. Haymore, A. van Hasselt, R. Beck, Annals of the New York Acad. Sci., 415, 1983, S.159-175).

Über die unterschiedlichen Prozesse und Katalysatoren sind eine Vielzahl von Olefinen für die Hydroformylierung zugänglich (siehe P. W. N. M. van Leeuwen, in Rhodium Catalyzed Hydroformylation, P. W. N. M. van Leeuwen, C. Claver (Hrsg.), Kluwer, Dordrecht, 2000). Technische Olefingemische, die als Edukte für die Oxo-Synthese verwendet werden, enthalten oftmals Olefine der verschiedensten Strukturen mit unterschiedlichen Verzweigungsgraden, unterschiedlicher Lage der Doppelbindung im Molekül und ggf. auch mit einer unterschiedlichen Kohlenstoffanzahl. Besonders gilt dies für Olefingemische, die durch Di-, Tri- oder weitgehende Oligomerisierung von Olefinen entstanden sind. Als Beispiele für technische Olefingemische, die durch Hydroformylierung zu den entsprechenden Aldehydgemischen umgesetzt werden, seien Tri- und Tetrapropen sowie Di-, Tri- und Tetrabuten genannt.

Die oben genannten technischen Olefingemische enthalten oftmals nur geringe Anteile an Olefinen mit endständigen Doppelbindungen. Um hieraus Produkte herzustellen, in denen mehr terminal hydroformyliertes Aldehyd vorliegt als im ursprünglichen Olefingemisch, muss unter isomerisierenden Bedingungen hydroformyliert werden.

Geeignete Verfahren dafür sind beispielsweise Hochdruck-Hydroformylierungen mit Cobaltkatalysatoren. Diese Verfahren haben aber u.a. den Nachteil, dass relativ viele Nebenprodukte wie Alkane, Acetale und Ether gebildet werden und sehr drastische Reaktionsbedingungen (hohe Temperatur, hoher Druck) notwendig sind (siehe auch Klaus-Diether Wiese, Dietmar Obst, Top. Organomet. Chem. 2006, 18, 1-33).

Bei der Verwendung von Rhodiumkomplexen als Katalysator ist der Ligand für die Produktzusammensetzung der Aldehyde mitbestimmend. Nichtmodifizierte Rhodiumcarbonylkomplexe katalysieren die Hydroformylierung von Olefinen mit end- und innenständigen Doppelbindungen, wobei die Olefine auch verzweigt sein können, zu Aldehyden mit einem hohen Verzweigungsgrad. Der Anteil an terminal hydroformyliertem Olefin ist im Vergleich zum Cobalt-katalysierten Produkt deutlich geringer.

Die Hydroformylierung von Olefinen mit innenständigen Doppelbindungen an Katalysatorsystemen, die sterisch anspruchsvolle Bisphosphitliganden enthalten, erfolgt bei langkettigen Olefinen zwar mit guter Selektivität, jedoch nicht mit einer zufriedenstellenden Aktivität (P. W. N. M. van Leeuwen, in Rhodium Catalyzed Hydroformylation, P. W. N. M. van Leeuwen, C. Claver (Hrsg.), Kluwer, Dordrecht, 2000).

In Angew. Chem. Int. Ed. 2000, 39, No. 9, S.1639-1641 von Börner et al. werden Phosphonite in der Hydroformylierung eingesetzt, also Liganden, welche eine P-C- und zwei P-O-Bindungen aufweisen. Die hier beschriebenen Phosphonite weisen bei einem Einsatz in der Hydroformylierung n/iso-Selektivität (n/iso = das Verhältnis von linearem Aldehyd (= n) zu verzweigtem (= iso) Aldehyd)) von 0,61 bis 1,57 auf.
Allerdings ist die Herstellung dieser Liganden auf Basis einer Phosphonitstruktur im Falle einer großtechnischen Synthese deutlich aufwändiger als beispielsweise die Herstellung von Phosphitliganden. Dieser Punkt spielt insbesondere bei dem Einsatz dieser Liganden in einem großtechnischen Prozess eine elementare Rolle. Die Synthese der als Liganden eingesetzten Verbindungen sollte so günstig und einfach wie möglich sein.

Rhodium-Monophosphit-Komplexe in katalytisch aktiven Zusammensetzungen hingegen sind geeignet für die Hydroformylierung von verzweigten Olefinen mit innenständigen Doppelbindungen.
Seit den 1970er Jahren ist die Verwendung von so genannten "bulky phosphites" in der Hydroformylierung beschrieben (siehe u.a. van Leeuwen et. al, Journal of Catalysis, 2013, 298, 198-205). Diese zeichnen sich durch eine gute Aktivität aus, jedoch ist die n/i-Selektivität für endständig hydroformylierte Verbindungen verbesserungswürdig.

Neben dem Einsatz von reinen Liganden ist auch der Einsatz von Ligandenmischungen in der Literatur beschrieben.

In US 20120253080 wird der Einsatz von Monophosphiten mit Bisphosphiten beschrieben. Jedoch hat diese Kombination den Nachteil, dass die Bisphosphite zwar eine gute Selektivität besitzen, im Fall von langkettigen Olefinen ist ihre Aktivität jedoch sehr gering und somit verbesserungswürdig. In einem großtechnischen Prozess ist neben der

Selektivität zum gewünschten Produkt auch die Raum-Zeit-Ausbeute bzw. die Aktivität des Katalysatorsystems ein wichtiger Faktor im Hinblick auf dessen Rentabilität. Außerdem sind die Bisphosphite häufig in ihrer Herstellung deutlich teurer als beispielsweise Monophosphite.

In EP 1 099 678 wird der Einsatz von Phosphoniten mit Bisphosphiten beschrieben. Hierbei ist jedoch nachteilig, dass beide Ligandentypen in ihrer Herstellung sehr teuer sind, und ein großtechnischer Prozess somit kaum wirtschaftlich sein kann. Außerdem beeinflusst der Zusatz des Bisphosphitliganden die Ausbeute der Reaktion merklich, da diese Liganden bei der Verwendung von z.B. Dibuten als Substrat weniger aktiv sind.

Es ist daher wünschenswert, ein Katalysatorsystem zu entwickeln, das die im Stand der Technik gezeigten Nachteile nicht aufweist.

Aufgabe der vorliegende Erfindung war es daher, ein Katalysatorsystem zur Hydroformylierung von Olefinen bereitzustellen, mit dem verzweigte, unverzweigte, end- und innenständige Olefine mit hohen Ausbeuten und Selektivitäten terminal hydroformyliert werden könne, d.h. möglichst lineare Aldehyde hergestellt werden können.
Des Weiteren soll das Verhältnis Kosten / Nutzen der zum Einsatz kommenden Liganden optimiert werden.

Die Aufgabe wird gelöst durch ein Gemisch nach Anspruch 1.

Gemisch umfassend mindestens eine der beiden Verbindungen gemäß der Strukturen Ia oder **Ib:**

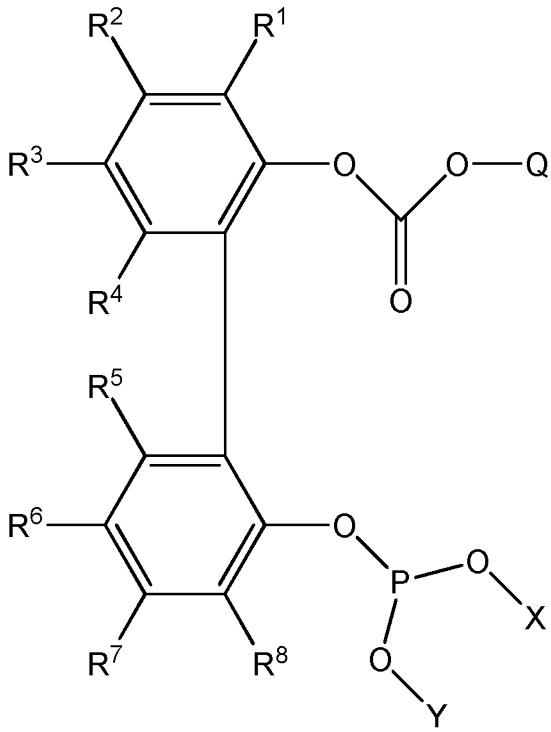

**Ia**

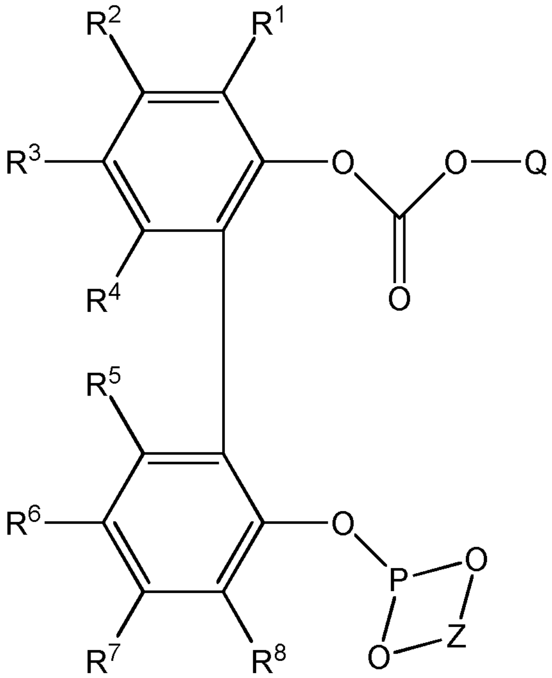

**Ib**

wobei

$R^1$, $R^2$, $R^3$, $R^4$, $R^5$, $R^6$, $R^7$, $R^8$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, Halogen, COO-($C_1$-$C_{12}$)-Alkyl, CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl,-COOH, -OH, -$SO_3H$ -$SO_3Na$ -$NO_2$, -CN, -$NH_2$, -N[($C_1$-$C_{12}$)-Alkyl]$_2$;
X und Y jeweils unabhängig voneinander ausgewählt sind aus: -($C_1C_{12}$)-Alkyl, -($C_6$-$C_{20}$)-Aryl, -($C_6$-$C_2$0)-Aryl-($C_1$-$C_{12}$)-Alkyl, -($C_6$-$C_{20}$)-Aryl-O-($C_1$-$C_{12}$)-Alkyl, -($C_1$-$C_{12}$)-Alkyl-($C_6$-$C_{20}$)-Aryl, -($C_6$-$C_{20}$)-Aryl-COO-($C_1$-$C_{12}$)-Alkyl, -($C_6$-$C_2$o)-Aryl-CONH-($C_1$-$C_{12}$)-Alkyl, -($C_6$-$C_2$o)-Aryl-CON[($C_1$-$C_{12}$)-Alkyl]$_2$, -($C_4$-$C_{20}$)-Heteroaryl, -($C_4$-$C_2$o)-Heteroaryl-($C_1$-$C_{12}$)-Alkyl, -($C_5$-$C_8$)-Cycloalkyl-($C_4$-$C_{20}$)-Aryl-CO-($C_6$-$C_{20}$)-Aryl,
Z ausgewählt ist aus: -($C_1$-$C_{12}$)-Alkyl-, -($C_6$-$C_{20}$)-Aryl-, -($C_6$-$C_{20}$)-Aryl-($C_1$-$C_{12}$)-Alkyl-, -($C_1$-$C_{12}$)-Alkyl-($C_6$-$C_{20}$)-Aryl-, -($C_4$-$C_{20}$)-Heteroaryl-, -($C_6$-$C_{20}$)-Aryl-CO-($C_6$-$C_{20}$)-Aryl-, -($C_6$-$C_{20}$)-Aryl-($C_6$-$C_{20}$)-Aryl-;
Q ausgewählt ist aus: -($C_1$-$C_{18}$)-Alkyl-, -($C_1$-$C_{12}$)-Alkyl-($C_1$-$C_{20}$)-Aryl-, -($C_1$-$C_{18}$)-Halogenalkyl-,-NH-($C_1$-$C_{18}$)-Alkyl, wobei die genannten Alkyl-, Heteroalkyl-, Cycloalkyl-, Heterocycloalkyl-, Aryl- und Heteroarylgruppen substituiert sein können;
sowie eine Verbindung gemäß der Struktur **IIa**:

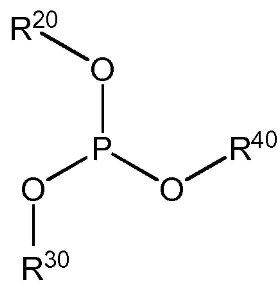

**IIa**

wobei

$R^{20}$, $R^{30}$, $R^{40}$ jeweils unabhängig voneinander ausgewählt sind aus: -($C_1$-$C_{12}$)-Alkyl, -($C_6$-$C_{20}$)-Aryl, -($C_3$-$C_{12}$)-Cyloalkyl,

außerdem können zwei Reste $R^{20}$ und $R^{30}$ oder $R^{20}$ und $R^{40}$ oder $R^{30}$ und $R^{40}$ miteinander verbrückt sein, und eine -($C_6$-$C_{20}$)-Aryl-($C_6$-$C_{20}$)-Aryl-Einheit aufweisen,
wobei die genannten Alkyl-, Cycloalkyl-, Arylgruppen substituiert sein können.

Mit einem solchen Liganden-Gemisch ist es möglich, verzweigte, unverzweigte, end- und innenständige Olefine mit hohen Ausbeuten und Selektivitäten terminal zu hydroformylieren, d.h. lineare Aldehyde herzustellen.
Durch den Einsatz des erfindungsgemäßen Liganden-Gemisches kann die Selektivität des Produktes gezielt gesteuert werden.

($C_1$-$C_2$)-Alkyl und O-($C_1$-$C_2$)-Alkyl können jeweils unsubstituiert oder durch einen oder mehrere gleiche oder verschiedene Reste substituiert sein, die ausgewählt sind unter ($C_3$-$C_{12}$)-Cycloalkyl, ($C_3$-$C_{,2}$)-Heterocycloalkyl, ($C_6$-$C_{20}$)-Aryl, Fluor, Chlor, Cyano, Formyl, Acyl oder Alkoxycarbonyl.

($C_3$-$C_{,2}$)-Cycloalkyl und ($C_3$-$C_{12}$)-Heterocycloalkyl können jeweils unsubstituiert oder durch einen oder mehrere gleiche oder verschiedene Reste substituiert sein, die ausgewählt sind unter ($C_1$-$C_2$)-Alkyl, ($C_1$-$C_{12}$)-Alkoxy, ($C_3$-$C_{12}$)-Cycloalkyl, ($C_3$-$C_{12}$)-Heterocycloalkyl, ($C_6$-$C_{20}$)-Aryl, Fluor, Chlor, Cyano, Formyl, Acyl oder Alkoxycarbonyl.

($C_6$-$C_{20}$)-Aryl und -($C_6$-$C_{20}$)-Aryl-($C_6$-$C_{20}$)-Aryl- können jeweils unsubstituiert oder durch einen oder mehrere gleiche oder verschiedene Reste substituiert sein, die ausgewählt sind unter -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$; -$SO_3Na$, -$NO_2$, -CN,-$NH_2$, -N[($C_1$-$C_{12}$)-Alkyl]$_2$.

Im Rahmen der Erfindung umfasst der Ausdruck -($C_1$-$C_{12}$)-Alkyl geradkettige und verzweigte Alkylgruppen. Vorzugsweise handelt es sich dabei um unsubstituierte geradkettige oder verzweigte -($C_1$-$C_8$)-Alkyl- und ganz bevorzugt -($C_1$-$C_6$)-Alkylgruppen. Beispiele für -($C_1$-$C_{12}$)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -($C_1$-$C_{12}$)-Alkyl gelten auch für die Alkylgruppen in -O-($C_1$-$C_{12}$)-Alkyl, also in -($C_1$-$C_{12}$)-Alkoxy. Vorzugsweise handelt es sich dabei um unsubstituierte geradkettige oder verzweigte -($C_1$-$C_6$)-Alkoxygruppen.

Substituierte -($C_1$-$C_{12}$)-Alkylgruppen und substituierte -($C_1$-$C_{12}$)-Alkoxygruppen können in Abhängigkeit von ihrer Kettenlänge, einen oder mehrere Substituenten aufweisen. Die Substituenten sind vorzugsweise unabhängig voneinander ausgewählt unter -($C_3$-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl.

Der Ausdruck -($C_3$-$C_{12}$)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Der Ausdruck -($C_3$-$C_{12}$)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -($C_3$-$C_{12}$)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1,2,3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -($C_3$-$C_{12}$)-Heterocycloalkylgruppen Tetrahydrothiophenyl, Tetrahydrofuryl, Tetrahydropyranyl und Dioxanyl.

Substituierte -($C_3$-$C_{12}$)-Cycloalkylgruppen und substituierte -($C_3$-$C_{12}$)-Heterocycloalkylgruppen können, in Abhängigkeit von ihrer Ringgröße, einen oder mehrere (z.B. 1, 2, 3, 4 oder 5) weitere Substituenten aufweisen. Diese Substituenten sind vorzugsweise unabhängig voneinander ausgewählt unter -($C_1$-$C_{12}$)-Alkyl, -($C_1$-$C_{12}$)-Alkoxy, -($C_3$-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl. Substituierte -($C_3$-$C_{12}$)-Cycloalkylgruppen tragen vorzugsweise eine oder mehrere -($C_1$-$C_6$)-Alkylgruppen. Substituierte -($C_3$-$C_{12}$)-Heterocycloalkylgruppen tragen vorzugsweise eine oder mehrere -($C_1$-$C_6$)-Alkylgruppen.

Der Ausdruck -($C_6$-$C_{20}$)-Aryl und -($C_6$-$C_{20}$)-Aryl-($C_6$-$C_{20}$)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -($C_6$-$C_{10}$)-Aryl und -($C_6$-$C_{10}$)-Aryl-($C_6$-$C_{10}$)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -($C_6$-$C_{20}$)-Arylgruppen und -($C_6$-$C_{20}$)-Aryl-($C_6$-$C_{20}$)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Ha-

logen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$; - $SO_3Na$, -$NO_2$, -CN, -$NH_2$, -N[($C_1$-$C_{12}$)-Alkyl]$_2$.

Substituierte -($C_6$-$C_{20}$)-Arylgruppen und -($C_6$-$C_{20}$)-Aryl-($C_6$-$C_{20}$)-Arylgruppen sind vorzugsweise substituierte -($C_6$-$C_{10}$)-Arylgruppen und -($C_6$-$C_{10}$)-Aryl-($C_6$-$C_{10}$)-Arylgruppen, insbesondere substituiertes Phenyl oder substituiertes Naphthyl. Substituierte -($C_6$-$C_{20}$)-Arylgruppen tragen vorzugsweise eine oder mehrere z.B. 1, 2, 3, 4 oder 5 Substituenten, ausgewählt unter -($C_1$-$C_{12}$)-Alkylgruppen, -($C_1$-$C_{12}$)-Alkoxygruppen.

In einer Ausführungsform ist Q ausgewählt aus: -($C_1$-$C_{12}$)-Alkyl-, -($C_1$-$C_3$)-Alkyl-($C_1$-$C_6$)-Aryl-, -($C_1$-$C_{18}$)-Halogenalkyl-,-NH-($C_1$-$C_8$)-Alkyl.

In einer Ausführungsform umfasst das Gemisch mindestens eine der beiden Verbindungen gemäß der Strukturen **Ic** oder Id:

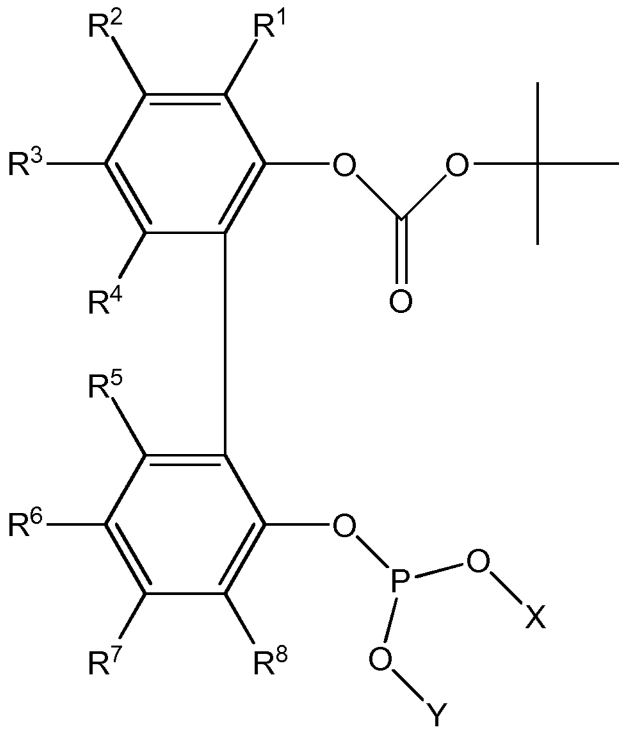

**Ic**

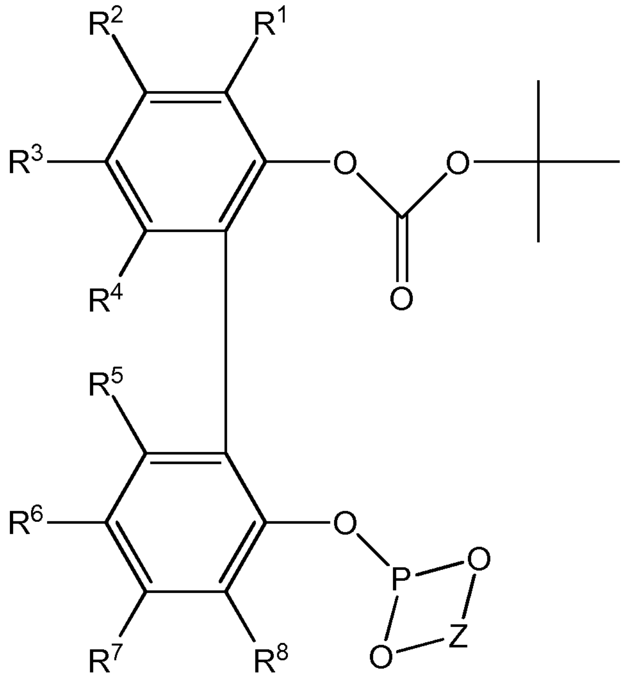

Id

In einer Ausführungsform sind X und Y jeweils unabhängig voneinander ausgewählt aus: $-(C_1-C_{12})$-Alkyl, $-(C_6-C_{20})$-Aryl, $-(C_6-C_{20})$-Aryl-$(C_1-C_{12})$-Alkyl, $-(C_6-C_{20})$-Aryl-O-$(C_1-C_{12})$-Alkyl, $-(C_6-C_{20})$-Aryl-COO-$(C_1-C_{12})$-Alkyl, $-(C_6-C_{20})$-Aryl-CONH-$(C_1-C_{12})$-Alkyl, $-(C_6-C_{20})$-Aryl-CON$[(C_1-C_{12})$-Alkyl$]_2$, $-(C_4-C_{20})$-Heteroaryl, $-(C_4-C_{20})$-Heteroaryl-$(C_1-C_{12})$-Alkyl.

In einer Ausführungsform sind X und Y jeweils unabhängig voneinander ausgewählt aus: $-(C_1-C_{12})$-Alkyl, $-(C_6-C_{20})$-Aryl, $-(C_6-C_{20})$-Aryl-$(C_1-C_{12})$-Alkyl, $-(C_6-C_{20})$-Aryl-O-$(C_1-C_{12})$-Alkyl, $-(C_6-C_{20})$-Aryl-COO-$(C_1-C_{12})$-Alkyl.

In einer Ausführungsform ist Z ausgewählt aus: $-(C_1-C_{12})$-Alkyl-, $-(C_6-C_{20})$-Aryl-, $-(C_6-C_{20})$-Aryl-$(C_1-C1_2)$-Alkyl-, $-(C_6-C_{20})$-Aryl-CO-$(C_6-C_{20})$-Aryl-, $-(C_1-C_{12})$-Alkyl-$(C_6-C_{20})$-Aryl-, $-(C_6-C_{20})$-Aryl-$(C_6-C_{20})$-Aryl-.

In einer Ausführungsform sind $R^1$, $R^2$, $R^3$, $R^4$, $R^5$, $R^6$, $R^7$, $R^8$ jeweils unabhängig voneinander ausgewählt aus: -H, $-(C_1-C_{12})$-Alkyl, -O-$(C_1-C_{12})$-Alkyl, -O-$(C_6-C_{20})$-Aryl, $-(C_6-C_{20})$-Aryl, -COO-$(C_1-C_{12})$-Alkyl, -CONH-$(C_1-C_{12})$-Alkyl, $-(C_6-C_{20})$-Aryl-CON$[(C_1-C_{12})$-Alkyl$]_2$, -CO-$(C_1-C_{12})$-Alkyl, -CO-$(C_6-C_{20})$-Aryl,-COOH, -OH, $-NH_2$, $-N[(C_1-C_{12})$-Alkyl$]_2$.

In einer Ausführungsform stehen X und Y für die gleichen Reste.

In einer Ausführungsform stehen $R^3$ und $R^6$ für -O-$(C_1-C_{12})$-Alkyl.

In einer Ausführungsform stehen $R^3$ und $R^6$ für -OMe.

In einer Ausführungsform stehen $R^1$ und $R^8$ für $-(C_1-C_{12})$-Alkyl.

In einer Ausführungsform stehen $R^1$ und $R^8$ für *tert*-Butyl.

In einer Ausführungsform umfasst das Gemisch eine Verbindung gemäß der Struktur Ie:

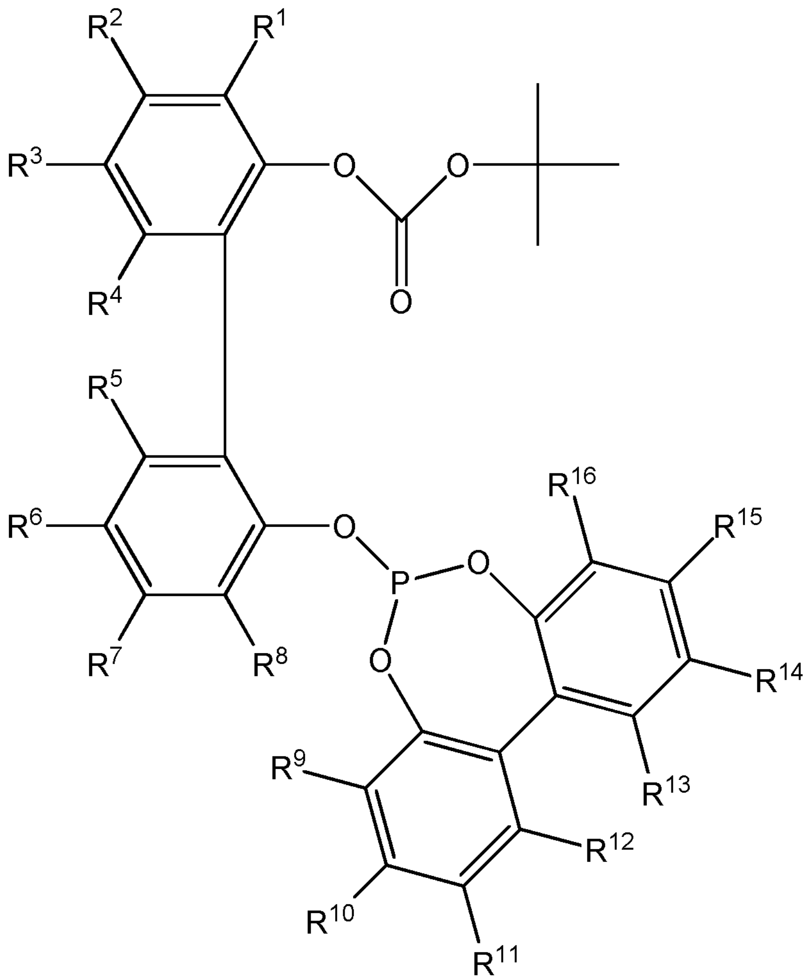

**Ie**

wobei $R^9$, $R^{10}$, $R^{11}$, $R^{12}$, $R^{13}$, $R^{14}$, $R^{15}$, $R^{16}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$; -$SO_3Na$, -$NO_2$, -CN, -$NH_2$, -N[($C_1$-$C_{12}$)-Alkyl]$_2$.

In einer Ausführungsform umfasst das Gemisch eine Verbindung gemäß der Struktur **If:**

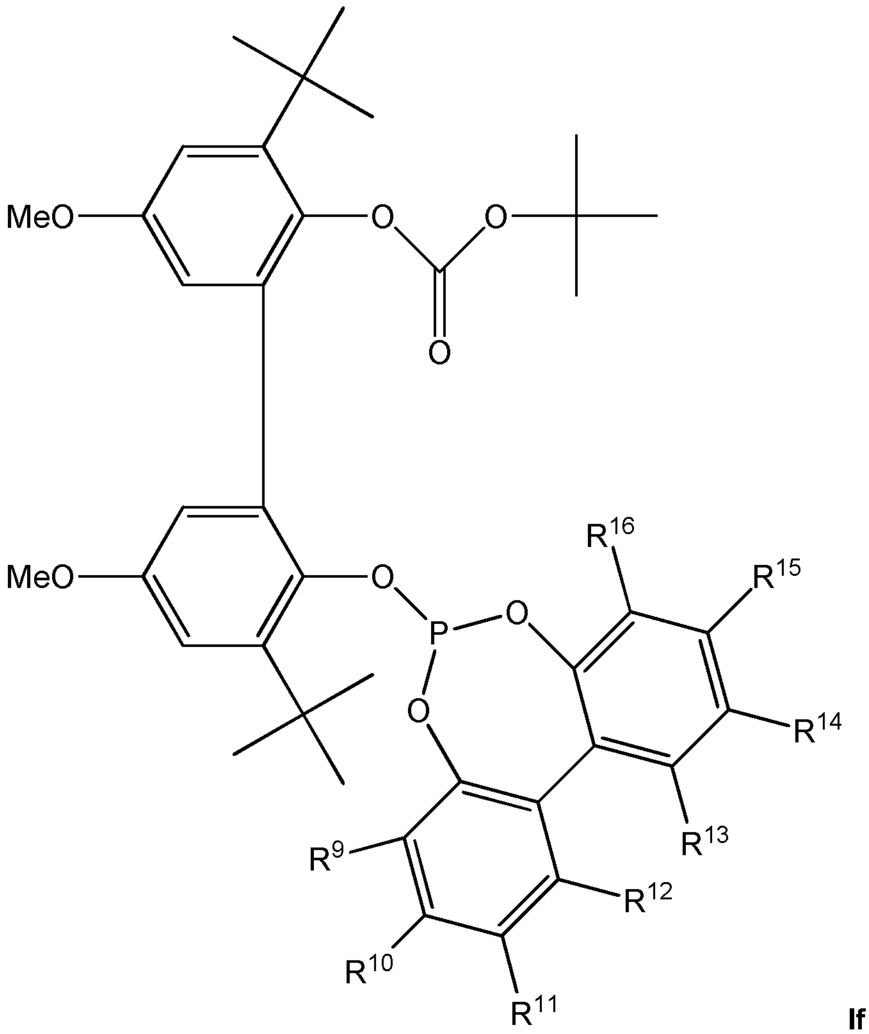

If

wobei $R^9$, $R^{10}$, $R^{11}$, $R^{12}$, $R^{13}$, $R^{14}$, $R^{15}$, $R^{16}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$, -$SO_3Na$, -$NO_2$, -CN, -$NH_2$, -N[($C_1$-$C_{12}$)-Alkyl]$_2$.

In einer Ausführungsform sind $R^{20}$, $R^{30}$, $R^{40}$ jeweils unabhängig voneinander ausgewählt aus: -($C_1$-$C_{12}$)-Alkyl, -($C_6$-$C_{20}$)-Aryl,

wobei die genannten Alkyl-, Arylgruppen substituiert sein können.

In einer Ausführungsform umfasst das Gemisch eine Verbindung gemäß der Struktur **IIb:**

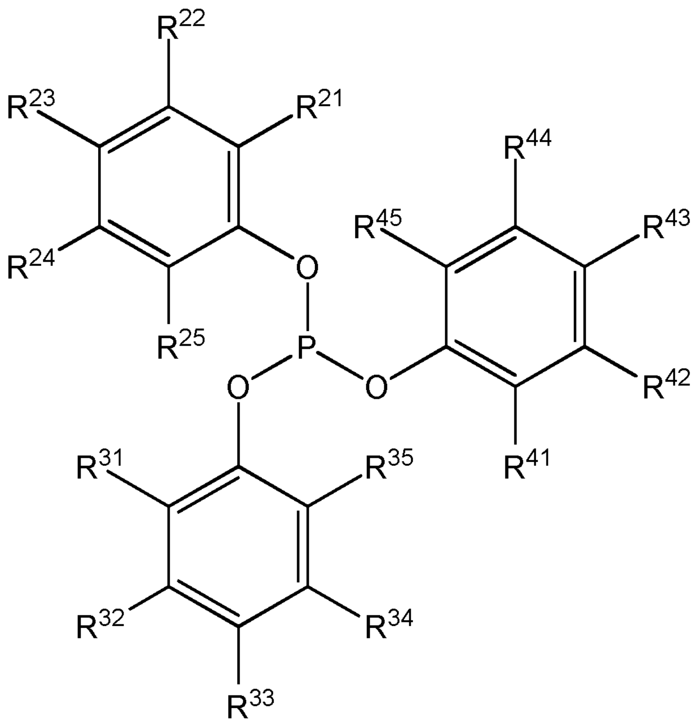

**IIb**

wobei

$R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$, -$SO_3Na$ -$NO_2$, -CN, -$NH_2$, -N[($C_1$-$C_{12}$)-Alkyl]$_2$.

In einer Ausführungsform sind $R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ jeweils unabhängig voneinander ausgewählt aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -$SO_3H$; -$SO_3Na$,-$NH_2$, -N[($C_1$-$C_{12}$)-Alkyl]$_2$.

In einer Ausführungsform sind $R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ jeweils unabhängig voneinander ausgewählt aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

In einer Ausführungsform stehen $R^{21}$, $R^{31}$, $R^{41}$ jeweils für *tert*-Butyl.

In einer Ausführungsform stehen $R^{23}$, $R^{33}$, $R^{43}$ jeweils für *tert*-Butyl.

In einer Ausführungsform stehen $R^{23}$, $R^{33}$, $R^{43}$ jeweils für Methyl.

Neben dem Gemisch wird auch ein Komplexgemisch beansprucht, welcher ein solches Gemisch aufweist.

Komplexgemisch umfassend:

- ein zuvor beschriebenes Gemisch,
- ein Metallatom ausgewählt aus: Rh, Ru, Co, Ir.

Bei dem Komplexgemisch können drei unterschiedliche Fälle vorliegen:

1) Das Komplexweißt entweder Liganden des Typs **I** oder **II** auf, und die Mischung besteht darin, dass Komplexmoleküle die nur Liganden des Typs **I** aufweisen mit Komplexmolekülen gemischt sind, die nur Liganden des Typs **II** aufweisen.

2) Ein Komplexweist an sich schon Liganden des Typs **I** und **II** auf.

3) Ist eine Mischform aus 1) und 2).

Neben den Gemischen / Komplexgemischen wird auch deren Verwendung als Komplexgemische zur Katalyse einer Hydroformylierungsreaktion beansprucht. Hierbei stellen die Verbindungen des Gemisches die Liganden des Komplexes dar. Die Liganden koordinieren an das Metall-Zentralatom. Der so erhaltene Ligand-Metall-Komplex bzw. die so erhaltenen Komplexgemische katalysieren dann die Hydroformylierungsreaktion.

Verwendung eines zuvor beschriebenen Gemisches in einem Komplexgemisch zur Katalyse einer Hydroformylierungsreaktion.

Des Weiteren wird auch die Hydroformylierungsreaktion beansprucht, in welcher die Gemische bzw. Komplexgemische zum Einsatz kommen.

Verfahren umfassend die Verfahrensschritte:

a) Vorlegen eines Olefins,
b) Zugabe eines zuvor beschriebenen Komplexgemisches,
oder eines zuvor beschriebenen Gemisches und einer Verbindung, welche ein Metallatom ausgewählt aus: Rh, Ru, Co, Ir aufweist,
c) Zuführen von $H_2$ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Ausführungsform ist das Metall Rh.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende $C_6$-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende $C_8$-Olefingemisch (Di-n-buten, Di-iso-buten), Nonene, 2- oder 3-Methyloctene, das bei der Trimerisierung von Propen anfallende $C_9$-Olefingemisch (Tripropen), Decene, 2-Ethyl-1-octen, Dodecene, das bei der Tetramerisierung von Propen oder der Trimerisierung von Butenen anfallende $C_{12}$-Olefingemisch (Tetrapropen oder Tributen), Tetradecene, Hexadecene, das bei der Tetramerisierung von Butenen anfallende $C_{16}$-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Gemische / Komplexgemische $\alpha$-Olefine, endständig verzweigte, innenständige und innenständig verzweigte Olefine hydroformyliert werden. Bemerkenswert ist dabei die hohe Ausbeute an endständig hydrofomyliertem Olefin, selbst wenn im Edukt nur ein geringer Anteil an Olefinen mit endständiger Doppelbindung vorhanden war.

Im Folgenden soll die Erfindung anhand von Ausführungsbeispielen näher erläutert werden.

**Allgemeine Arbeitsvorschriften**

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego, Christina Chai, Butterworth Heinemann (Elsevier), 6. Auflage, Oxford 2009).
Alle präparativen Arbeiten erfolgten in ausgeheizten Gefäßen. Die Charakterisierung der Produkte erfolgte mittels NMR-Spektroskopie. Chemische Verschiebungen ($\delta$) werden in ppm angegeben. Die Referenzierung der $^{31}$P-NMR-Signale erfolgte gemäß: $SR_{31P}= SR_{1H}*(BF_{31P}/ BF_{1H})=SR_{1H}*0{,}4048$. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).
Die Aufnahme von Kernresonanzspektren erfolgte mittels eines Bruker Avance 300 bzw. Bruker Avance 400, die gaschromatografische Analyse an Agilent GC 7890A.

Die erfindungsgemäßen Liganden lassen sich hierbei auf verschiedenen Wegen herstellen. Drei mögliche Wege sind in den nachfolgenden Schemata (A bis C) dargestellt.
Die dargestellten Reaktionswege sind nur exemplarisch und in einer stark vereinfachten Form dargestellt. So kann nach Bedarf in allen Schritten zusätzlich Base verwendet werden. Weiterhin können die in den einzelnen Synthesestufen genannten Basen durch andere, dem Fachmann bekannte und kommerziell verfügbare Basen, substituiert werden.

Reaktionsweg A:

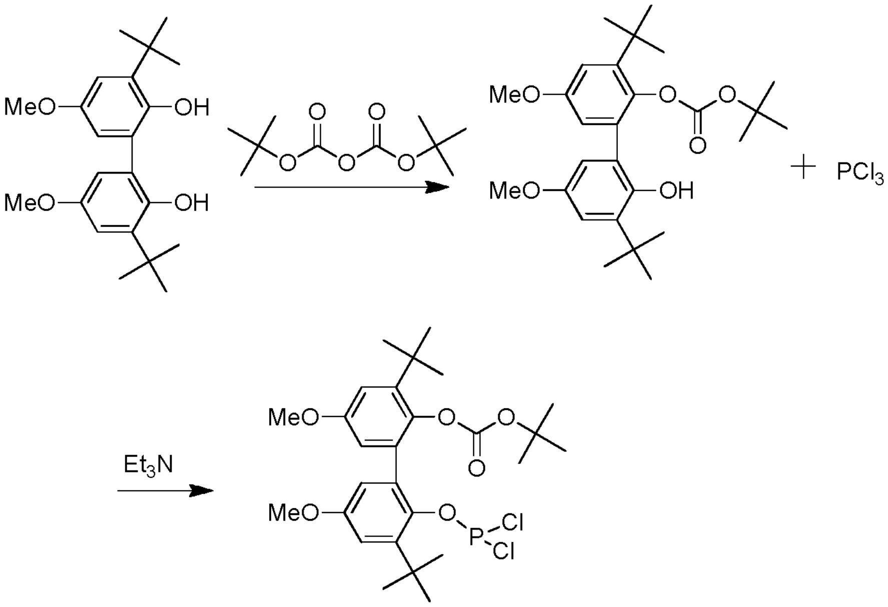


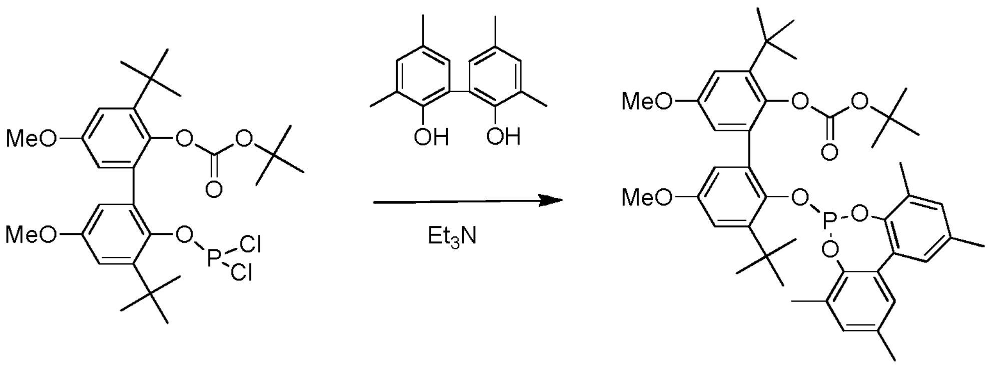


Reaktionsweg B:

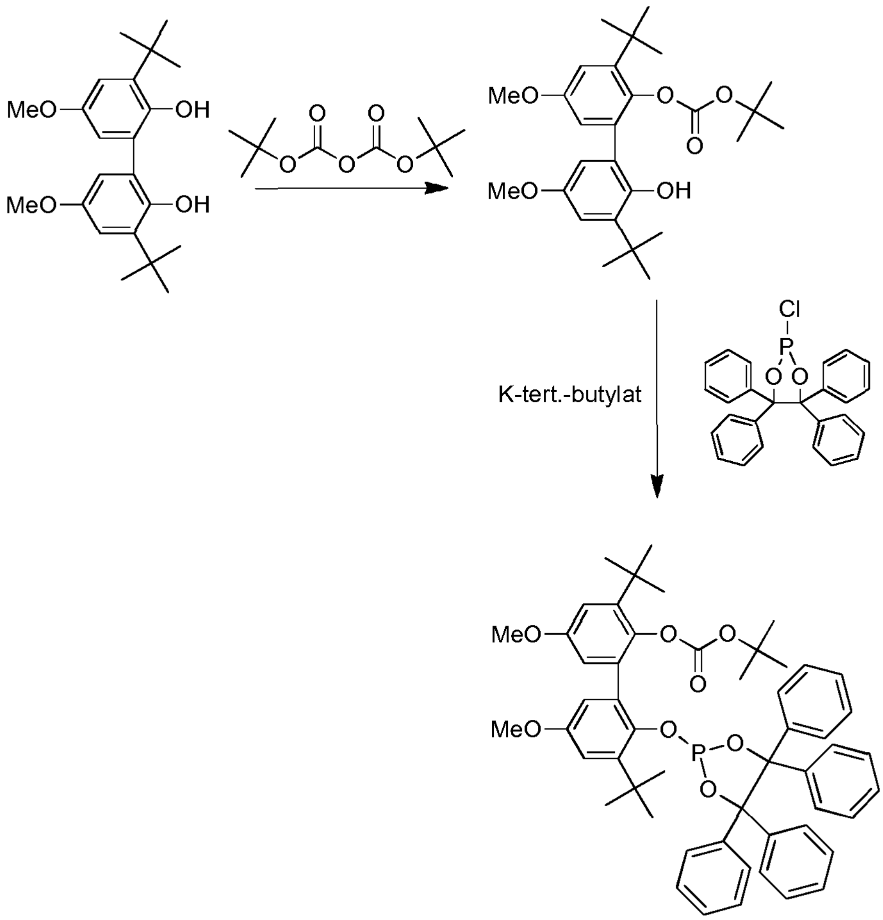


Reaktionsweg C:

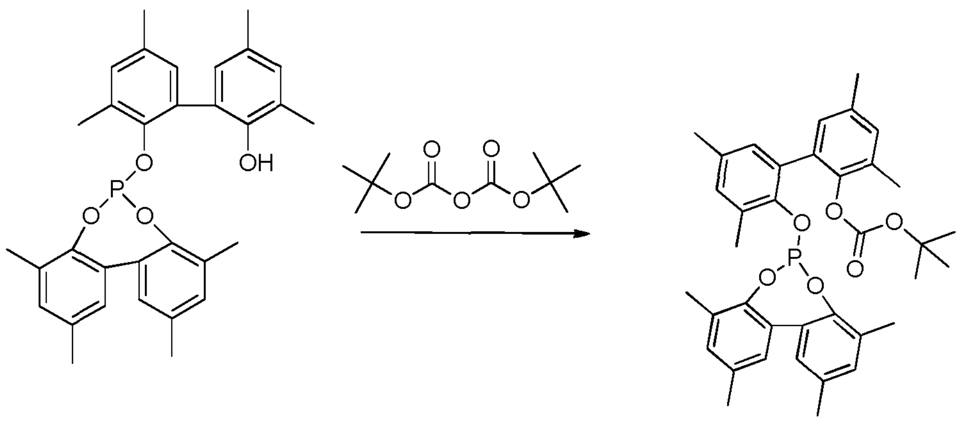

Beispiele für Liganden des Typs I:

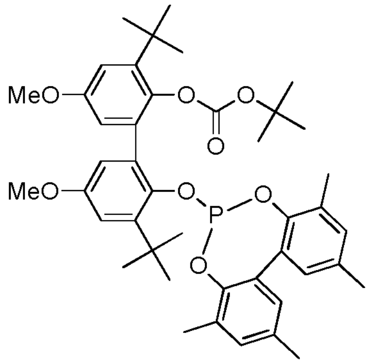

1

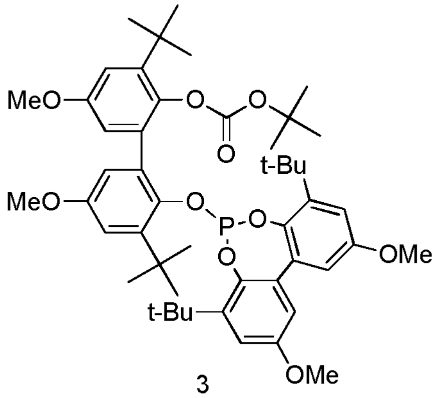

3

Synthese von Ligand 1

Reaktionsschema:

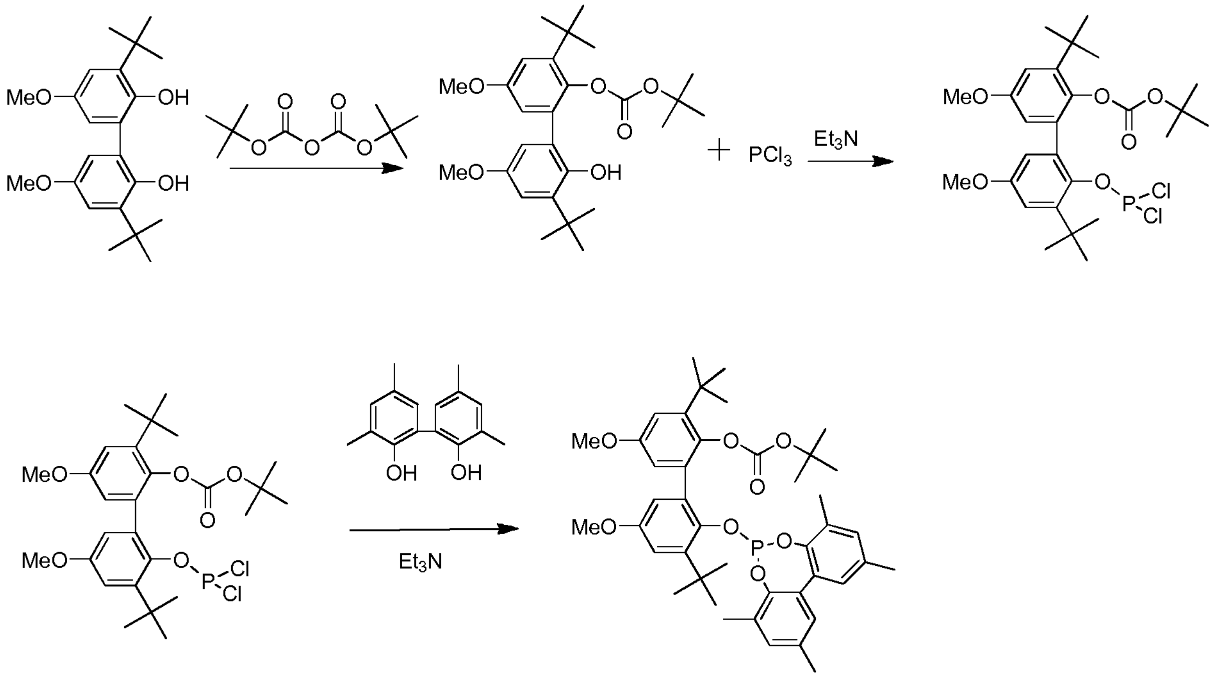


Einführung der BOC-Gruppe:

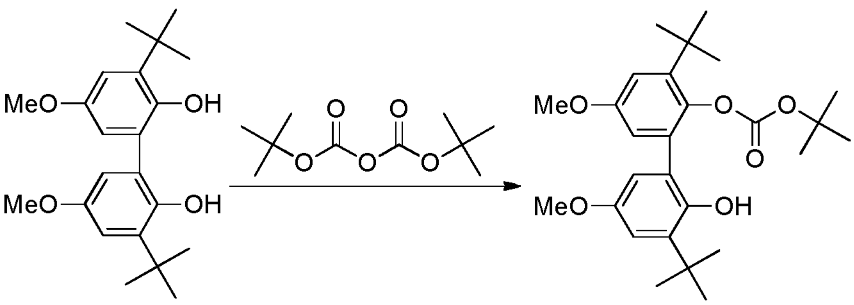

In einem 2L Schlenkkolben werden 400 mmol (143,8 g) des 3,3'-Di-tert.-butyl-5,5'-dimethoxy-[1,1'-biphenyl]-2,2'diol und 40 mmol (4,8 g) N,N-Dimethylaminopyridin (DMAP) in 900 mL $CH_2Cl_2$ gelöst. Anschließend wurden bei Raumtemperatur 400 mmol (88 g) Di-tert-butyldicarbonat in 280 ml $CH_2Cl_2$ gelöst, in einen 500 ml Tropftrichter überführt und innerhalb einer Stunde bei 32 °C zu der Biphenol/DMAP Lösung getropft. Die Lösung wurde über Nacht bei Raumtemperatur gerührt. Am nächsten Morgen wird das Lösungsmittel unter vermindertem Druck entfernt. Der leicht wachsartige, rötliche Rückstand wurde mit 800 ml n-Heptan versetzt und über Nacht gerührt. Dabei erhielt man einen weißen Rückstand, der abfiltriert, zweimal mit 50 ml n-Heptan nachgewaschen und dann getrocknet wurde. Das Zielprodukt konnte als weißer Feststoff (161,6 g, 84%) erhalten werden. $^1$H-NMR (Tolul-$d_8$): 95% und weitere Verunreinigungen.

Umsetzung von tert-Butyl-(3,3'-di-tert-butyl-2'-hydroxy-5,5'-dimethoxy-[1,1'-biphenyl]-2-yl)-carbonat mit Phosphortrichlorid:

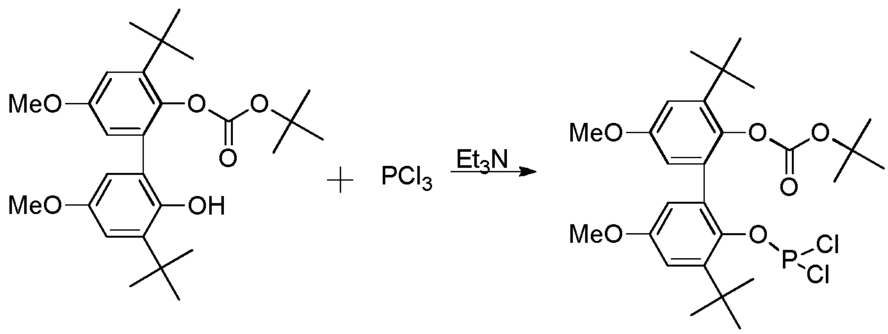


In einem sekurierten 250 ml Schlenkkolben wurden 12 g (0,026 mol) tert-Butyl-(3,3'-di-tert-butyl-2'-hydroxy-5,5'-dimethoxy-[1,1'-biphenyl]-2-yl)-carbonat unter Rühren in 120 ml getrocknetem Toluol und 12,8 ml (0,091 mol) Triethylamin gelöst.
In einem zweiten 500 ml Schlenkkolben wurden zunächst 100 ml getrocknetes Toluol mit 8,1 ml (0,091 mol) Phosphortrichlorid verrührt. Anschließend wurde die Phosphortrichlorid-Toluol-Lösung zu der zuvor hergestellten Carbonat-Amin-Toluol-Lösung innerhalb von 30 Minuten bei Raumtemperatur zugetropft. Nach vollständiger Zugabe wurde für 30 Minuten auf 80 °C erwärmt und über Nacht auf Raumtemperatur abgekühlt.
Am nächsten Morgen wurde die Mischung filtriert, mit 50 ml getrocknetem Toluol nachgewaschen und das Filtrat bis zur Trockne eingeengt. Das Zielprodukt konnte als Feststoff (13,1 g, 89%) erhalten werden. $^{31}$P-NMR (202,4 MHz, Toluol-$d_8$): 203,2 und 203,3 ppm (100%).

Umsetzung von tert-Butyl-(3,3'-di-tert-butyl-2'-((dichlorophosphino)oxy)-5,5'-dimethoxy-[1,1'-biphenyl]-2-yl)-carbonat mit 3,3',5,5'-Tetramethyl-(1,1'-biphenyl)-2,2'-diol

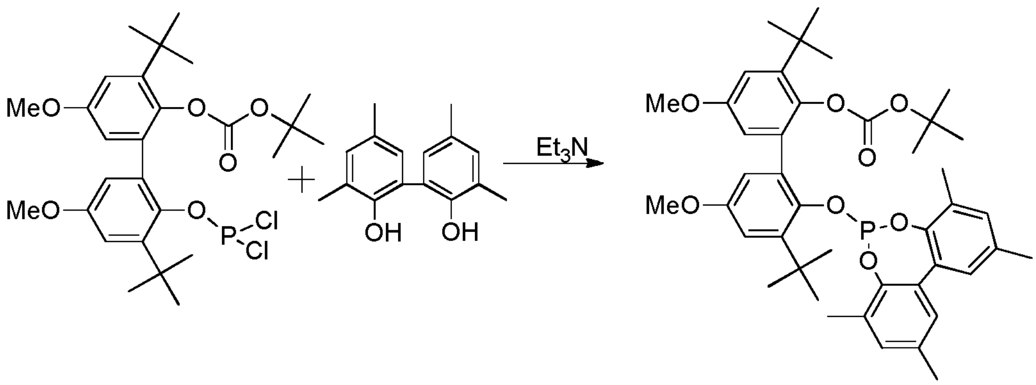


In einem sekurierten 1L Schlenkkolben wurden 24,7 g (0,044 mol) tert-Butyl-(3,3'-di-tert-butyl-2'-((dichlorophosphino)oxy)-5,5'-dimethoxy-[1,1'-biphenyl]-2-yl)-carbonat in 400 ml Acetonitril gelöst.

In einem zweiten sekurierten Schlenkkolben (1L) wurden 10,8 g (0,044 mol) 3,3',5,5'-Tetramethyl-(1,1'-biphenyl)-2,2'-diol in 200 ml Acetonitril und 13,1 ml (0,011 mol) getrocknetem Triethylamin unter Rühren gelöst. Anschließend wurde zu der Biphenol-Triethylamin-Lösung langsam die Chlorophosphit-Lösung hinzugetropft und über Nacht gerührt.
Der Ansatz wurde daraufhin filtriert und der Rückstand zweimal mit 15 ml Acetonitril gewaschen.
Das Filtrat wurde unter vermindertem Druck eingeengt bis ein Niederschlag ausfiel. Dieser wurde filtriert und getrocknet. Das Zielprodukt konnte als weißer Feststoff (28,5 g, 87%) erhalten werden. $^{31}$P-NMR (202,4 MHz,Toluol-$d_8$): 139,4 ppm (98,5%).

Synthese von Ligand 3

Umsetzung von tert-Butyl-(3,3'-di-tert-butyl-2'-((dichlorophosphino)oxy)-5,5'-dimethoxy-[1,1'-biphenyl]-2-yl)-carbonat mit 3,3-Di-tert.butyl-5.5-dimethoxy-biphenol

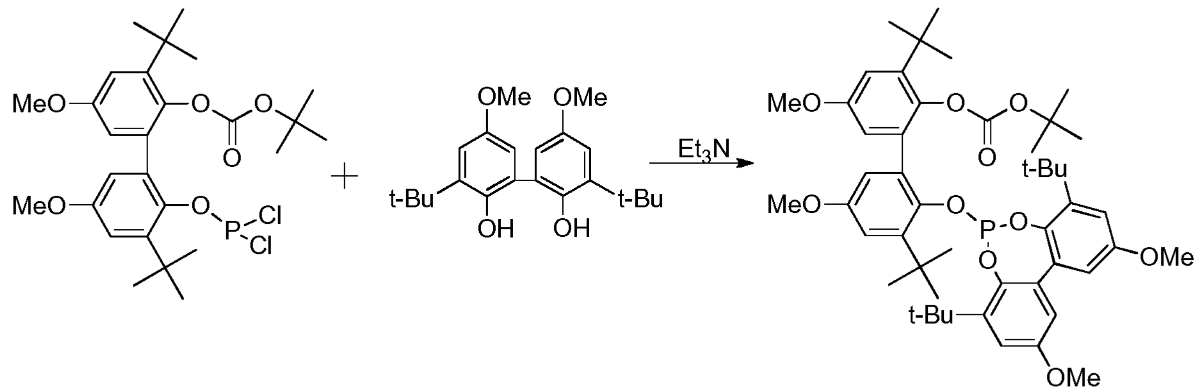


In einem sekurierten 250 ml Schlenkkolben wurden 7 g (0,0125 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.
In einem zweiten sekurierten Schlenkkolben (100 ml) wurden 4.5 g (0,0125 mol) 3,3-Di-tert.-butyl-5,5-dimethoxy-biphenol in 60 ml getrocknetem Acetonitril und 4.2 ml (0,03 mol) entgastem Triethylamin gelöst. Im Anschluss wurde die Biphenol-Triethylamin-Lösung langsam bei Raumtemperatur zu der Chlorophosphit-Lösung getropft und über Nacht bei Raumtemperatur gerührt.
Ein Teil des Lösungsmittels wurde unter vermindertem Druck entfernt. Der ausgefallene Feststoff wurde filtriert und getrocknet. Das Zielprodukt konnte als weißer Feststoff (10,5 g, 96%) erhalten werden. $^{31}$P-NMR (202,4 MHz, Toluol-$d_8$): 140,9 (95,2%) und weitere Verunreinigungen (weitere Verunreinigungen = P-H-Verbindungen, Oxidverbindungen, noch nicht vollständig umgesetztes Chlorophosphit).

**Durchführung der Katalyseversuche**

Versuchsbeschreibung - allgemein

In einem 100 ml-Autoklaven der Fa. Parr Instruments wurde bei 120 °C, 50 bar Synthesegasdruck ($CO/H_2$ = 1:1 (Vol-%)) n-Octene hydroformyliert. Als Precursor wurden bei einer Katalysatorkonzentration von 100 ppm Rh bezogen auf das gesamte Reaktionsgemisch 0.123 g $Rh(acac)(CO)_2$ vorgelegt. Als Lösemittel wurden jeweils 40 bis 46 g Toluol verwendet. Der Ligand 1 bzw. der Ligand 2 bzw. die Ligandenmischung bestehend aus den Liganden 1 und 2 wurde in unterschiedlichen molaren Überschüssen relativ zum Rhodium verwendet. Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.
Während der Reaktion wurde der Druck über eine Synthesegasregelung mit Massedurchflussmesser konstant gehalten. Die Rührerdrehzahl betrug 1200 $min^{-1}$. Proben wurden aus der Reaktionsmischung nach 12 Stunden gezogen. Die Ergebnisse der Versuche sind in Tabelle 1 zusammengefasst.

(acac = acetylacetonat)

In den Katalyseversuchen verwendete Liganden:

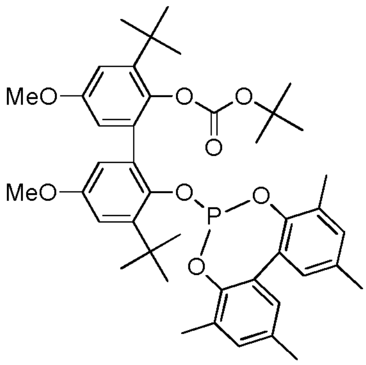

1

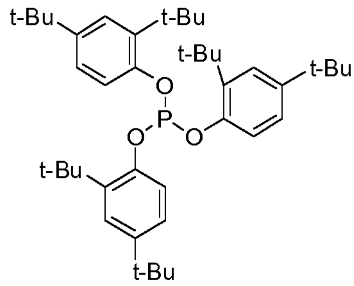

2

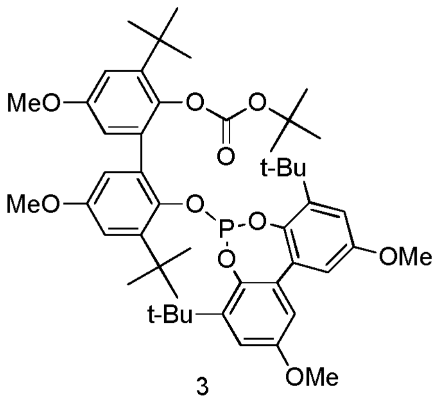

3

Die Herstellung der Liganden 1 und 3 ist im obigen experimentellen Teil beschreiben. Der Ligand 2 (TDTBPP bzw. Alkanox 240) ist kommerziell erhältlich.

Tabelle 1:

| (Ausbeute = Aldehyd und Alkohol Gesamtausbeute; S = Selektivität zum linearen Produkt) | | | | | | |
|---|---|---|---|---|---|---|
| Eintrag | T in [°C] | cRh in ppm | P/Rh Ligand 1 | P/Rh Ligand 2 | A (Aldehyde+Alkohole gesamt) in % | S (n-Nonanal) in % |
| 1 | 80 | 90 | 19,8 | 0 | 99,0 | 7,2 |
| 2 | 90 | 90 | 19,8 | 0 | 99,2 | 10,6 |
| 3 | 90 | 280 | 0 | 20,3 | 97,9 | 4,7 |
| 4 | 110 | 90 | 0 | 20,4 | 99,0 | 9,7 |
| 5* | 100 | 90 | 18,8 | 0,8 | 98,7 | 12,0 |
| 6* | 110 | 90 | 18,8 | 0,8 | 97,6 | 17,3 |
| 7* | 110 | 90 | 14,8 | 3,9 | 96,5 | 13,5 |
| 8* | 110 | 90 | 10,0 | 8,1 | 97,9 | 11,4 |
| 9* | 120 | 90 | 18,9 | 0,8 | 95,2 | 22,9 |
| 10* | 120 | 90 | 14,8 | 3,9 | 94,5 | 18,1 |
| 11* | 120 | 90 | 10,0 | 8,1 | 95,5 | 16,5 |
| 12* | 120 | 90 | 5,0 | 11,9 | 95,5 | 15,5 |

(fortgesetzt)

| (Ausbeute = Aldehyd und Alkohol Gesamtausbeute; S = Selektivität zum linearen Produkt) | | | | | | |
|---|---|---|---|---|---|---|
| Eintrag | T in [°C] | cRh in ppm | P/Rh Ligand 1 | P/Rh Ligand 2 | A (Aldehyde+Alkohole gesamt) in % | S (n-Nonanal) in % |
| 13* | 120 | 90 | 1,0 | 15,2 | 94,7 | 15,4 |
| * erfindungsgemäße Gemisch bzw. Komplexgemische<br>Reaktionsbedingungen: 50 bar Synthesegasdruck, Substrat: n-Octene | | | | | | |

Tabelle 1 enthält Versuche zur Hydroformylierung eines n-Octengemischs mit ca. 2 % 1-Octen, 40 % 2-Octenen, 36 % 3-Octenen und 23 % 4-Octenen. Innerhalb der Versuchsreihe wurden Mischungen der Liganden 1 und 2 mit unterschiedlichen molaren Verhältnissen untersucht. Bei den ersten vier Versuchen (Einträge 1 bis 4) handelt es sich um Vergleichsversuche. Hier kam jeweils nur einer der beiden Liganden, also entweder Ligand 1 oder Ligand 2, zum Einsatz.

Bei den Versuchen, welche mit erfindungsgemäßen Gemischen / Komplexgemischen durchgeführt wurden, konnten jeweils sehr gute Selektivitäten (S) erzielt werden.

Durch die Verwendung von erfindungsgemäßen Gemischen / Komplexgemischen, ist es möglich den Anteil an endständig hydroformylierten Produkt selektiv zu steuern. Die Selektivität zu den gewünschten linearen Aldehyden ist hierbei deutlich größer als beispielsweise bei dem kommerziell verfügbaren Liganden 2. Es ist hierbei besonders vorteilhaft, dass sich die beiden Liganden in der Mischung in ihrer Wirkung verstärken, und nur so viel von dem deutlich teureren Liganden 1 verwendet werden muss, wie nötig, um die erwünschte Produktselektivität zu erhalten. Dies stellt einen klaren wirtschaftlichen Vorteil gegenüber einer Verfahrensführung dar, welche ausschließliche mit dem Ligand 1 erfolgt.

In Tabelle 2 sind die Ergebnisse für die Hydroformylierung von Di-n-Buten angegeben. Di-n-Buten ist ein Gemisch aus Isomeren von n-Octenen (ca. 16 %), 3-Methyl-heptenen (ca. 65 %) und 3,4-Dimethylhexenen (ca. 19 %). (Ausbeute = Aldehyd und Alkohol Gesamtausbeute; S = Selektivität zum linearen Produkt)

Tabelle 2:

| Eintrag | Ligand A | Ligand B | P/Rh Ligand A | P/Rh Ligand B | A in % | S in % |
|---|---|---|---|---|---|---|
| 1 | - | 2 | - | 20 | 96,9 | 21,8 |
| 2* | 1 | 2 | 14,8 | 5,1 | 82,6 | 27,2 |
| 3* | 3 | 2 | 17,4 | 2,0 | 95,4 | 27,7 |
| 4* | 3 | 2 | 15,7 | 5,1 | 97,0 | 27,5 |
| 5* | 3 | 2 | 10,2 | 10,1 | 96,9 | 25,0 |
| 6* | 3 | 2 | 5,0 | 14,7 | 96,0 | 23,8 |
| * erfindungsgemäße Gemisch bzw. Komplexgemische | | | | | | |

Reaktionsbedingungen: 50 bar Synthesegasdruck, T = 140°C, Substrat: Di-n-buten, P : Rh = 20:1; 100 ppm [Rh]

Tabelle 2 enthält Versuche zur Hydroformylierung von Di-n-buten mit verschiedenen Gemischen / Komplexgemischen. Eintrag 1 zeigt einen Vergleichsversuch, welcher nur mit dem Ligand 2 durchgeführt wurde. Hier wurde zwar eine gute Ausbeute erzielt, jedoch lässt die Selektivität zu wünschen übrig.

Durch den Einsatz der erfindungsgemäßen Gemische / Komplexgemische konnte die Selektivität in allen Fällen gesteigert werden. Die Selektivität zu den gewünschten linearen Aldehyden ist hierbei merklich größer als bei dem kommerziell verfügbaren Liganden 2.

Durch die Verwendung der erfindungsgemäßen Gemische / Komplexgemische, ist es möglich den Anteil an endständig hydroformylierten Produkt selektiv zu steuern und zu steigern.

Der sehr teure Ligand 1 kann teilweiße durch den billigeren Ligand 2 ersetzt werden, was einen nennenswerten kommerziellen Vorteil darstellt. Das Verhältnis Kosten / Nutzen der eingesetzten Liganden wurde hierdurch deutlich verbessert.

Somit konnte mit Hilfe der obigen Beispiele gezeigt werden, dass die gestellten Aufgaben durch den Einsatz der erfindungsgemäßen Gemische / Komplexgemische gelöst wurden.

**Patentansprüche**

**1.** Gemisch umfassend mindestens eine der beiden Verbindungen gemäß der Strukturen **Ia** oder **Ib**:

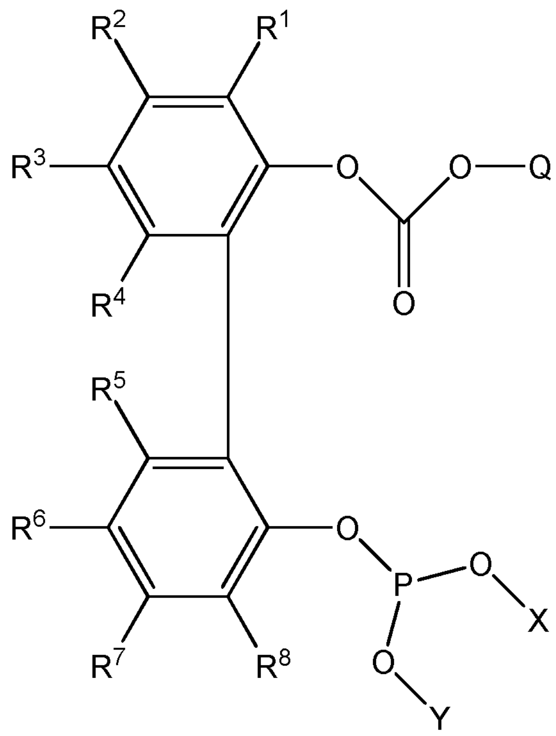

**Ia**

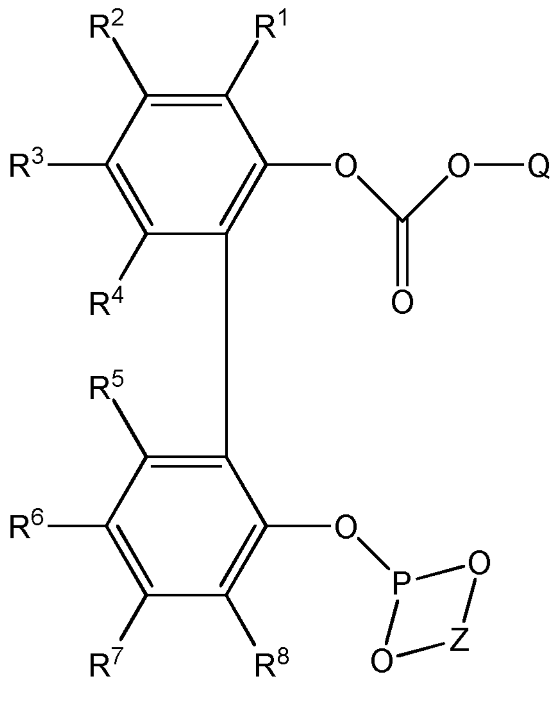

**Ib**

wobei

$R^1$, $R^2$, $R^3$, $R^4$, $R^5$, $R^6$, $R^7$, $R^8$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, Halogen, COO-($C_1$-$C_{12}$)-Alkyl, CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl,-COOH, -OH, -$SO_3H$ -$SO_3Na$, -$NO_2$, -CN, -$NH_2$, -N[($C_1$-$C_{12}$)-Alkyl]$_2$;

X und Y jeweils unabhängig voneinander ausgewählt sind aus: -($C_1$-$C_{12}$)-Alkyl, -($C_6$-$C_{20}$)-Aryl, -($C_6$-$C_{20}$)-Aryl-($C_1$-$C_{12}$)-Alkyl, -($C_6$-$C_{20}$)-Aryl-O-($C_1$-$C_{12}$)-Alkyl, -($C_1$-$C_{12}$)-Alkyl-($C_6$-$C_{20}$)-Aryl, -($C_6$-$C_{20}$)-Aryl-COO-($C_1$-$C_{12}$)-Alkyl, -($C_6$-$C_{20}$)-Aryl-CONH-($C_1$-$C_{12}$)-Alkyl, -($C_6$-$C_{20}$)-Aryl-CON[($C_1$-$C_{12}$)-Alkyl]$_2$, -($C_4$-$C_{20}$)-Heteroaryl, -($C_4$-$C_{20}$)-Heteroaryl-($C_1$-$C_{12}$)-Alkyl, -($C_5$-$C_8$)-Cycloalkyl-($C_4$-$C_{20}$)-Aryl-CO-($C_6$-$C_{20}$)-Aryl,

Z ausgewählt ist aus: -($C_1$-$C_{12}$)-Alkyl-, -($C_6$-$C_{20}$)-Aryl-, -($C_6$-$C_{20}$)-Aryl-($C_1$-$C_{12}$)-Alkyl-, -($C_1$-$C_{12}$)-Alkyl-($C_6$-$C_{20}$)-Aryl-, -($C_4$-$C_{20}$)-Heteroaryl-, -($C_6$-$C_{20}$)-Aryl-CO-($C_6$-$C_{20}$)-Aryl-, -($C_6$-$C_{20}$)-Aryl-($C_6$-$C_{20}$)-Aryl-;

Q ausgewählt ist aus: -($C_1$-$C_{18}$)-Alkyl-, -($C_1$-$C_{12}$)-Alkyl-($C_1$-$C_{20}$)-Aryl-, -($C_1$-$C_{18}$)-Halogenalkyl-,-NH-($C_1$-$C_{18}$)-Alkyl, wobei die genannten Alkyl-, Heteroalkyl-, Cycloalkyl-, Heterocycloalkyl-, Aryl- und Heteroarylgruppen substituiert sein können,

sowie eine Verbindung gemäß der Struktur **IIa:**

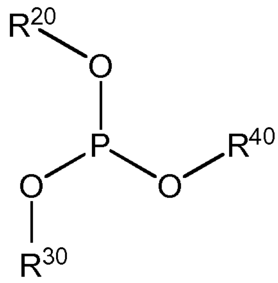

**IIa**

wobei

$R^{20}$, $R^{30}$, $R^{40}$ jeweils unabhängig voneinander ausgewählt sind aus: -($C_1$-$C_{12}$)-Alkyl, -($C_6$-$C_{20}$)-Aryl, -($C_3$-$C_{12}$)-Cycloalkyl,

außerdem können zwei Reste $R^{20}$ und $R^{30}$ oder $R^{20}$ und $R^{40}$ oder $R^{30}$ und $R^{40}$ miteinander verbrückt sein, und eine -($C_6$-$C_{20}$)-Aryl-($C_6$-$C_{20}$)-Aryl-Einheit aufweisen,

wobei die genannten Alkyl-, Cycloalkyl-, Arylgruppen substituiert sein können.

**2.** Gemisch nach Anspruch 1,
wobei Q ausgewählt ist aus: -($C_1$-$C_{12}$)-Alkyl-, -($C_1$-$C_3$)-Alkyl-($C_1$-$C_6$)-Aryl-, -($C_1$-$C_{18}$)-Halogenalkyl-,-NH-($C_1$-$C_a$)-Alkyl.

**3.** Gemisch nach einem der Ansprüche 1 oder 2,
umfassend mindestens eine der beiden Verbindungen gemäß einer der Strukturen **Ic** oder **Id:**

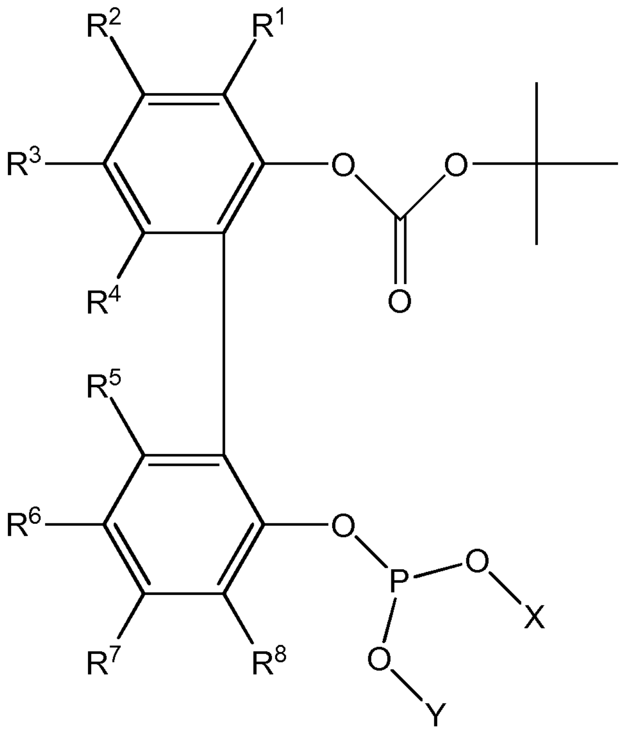

Ic

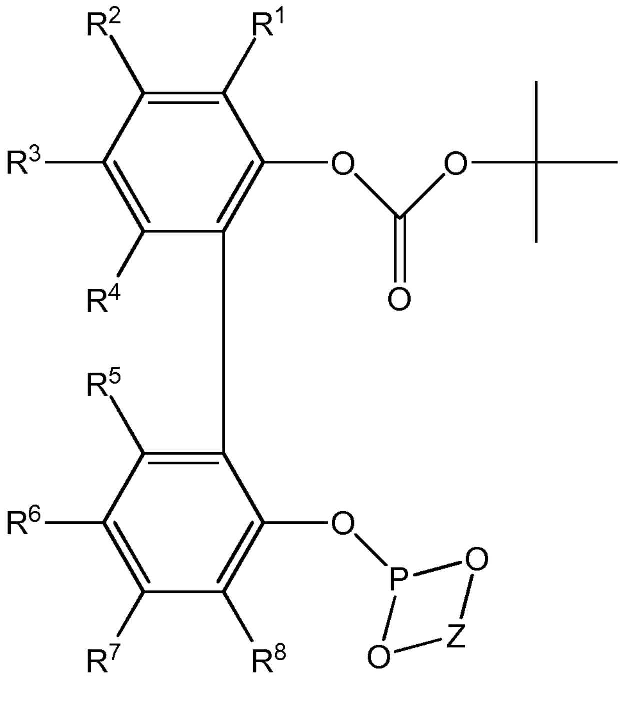

Id

**4.** Gemisch nach einem der Ansprüche 1 bis 3,
wobei X und Y jeweils unabhängig voneinander ausgewählt sind aus: -($C_1$-$C_{12}$)-Alkyl, -($C_6$-$C_{20}$)-Aryl, -($C_6$-$C_{20}$)-Aryl-($C_1$-$C_{12}$)-Alkyl, -($C_6$-$C_{20}$)-Aryl-O-($C_1$-$C_{12}$)-Alkyl, -($C_6$-$C_{20}$)-Aryl-COO-($C_1$-$C_{12}$)-Alkyl.

**5.** Gemisch nach einem der Ansprüche 1 bis 4,
wobei Z ausgewählt ist aus: -($C_1$-$C_{12}$)-Alkyl-, -($C_6$-$C_{20}$)-Aryl-, -($C_6$-$C_{20}$)-Aryl-($C_1$-$C_{12}$)-Alkyl-, -($C_6$-$C_{20}$)-Aryl-

CO-($C_6$-$C_{20}$)-Aryl-, -($C_1$-$C_{12}$)-Alkyl-($C_6$-$C_{20}$)-Aryl-, -($C_6$-$C_{20}$)-Aryl-($C_6$-$C_{20}$)-Aryl-.

**6.** Gemisch nach einem der Ansprüche 1 bis 5, wobei $R^1$, $R^2$, $R^3$, $R^4$, $R^5$, $R^6$, $R^7$, $R^8$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl,-COOH, -OH, -$NH_2$, -N[($C_1$-$C_{12}$)-Alkyl]$_2$.

**7.** Gemisch nach einem der Ansprüche 1 bis 6, wobei X und Y für die gleichen Reste stehen.

**8.** Gemisch nach einem der Ansprüche 1 bis 7, umfassend eine Verbindung gemäß der Struktur **Ie:**

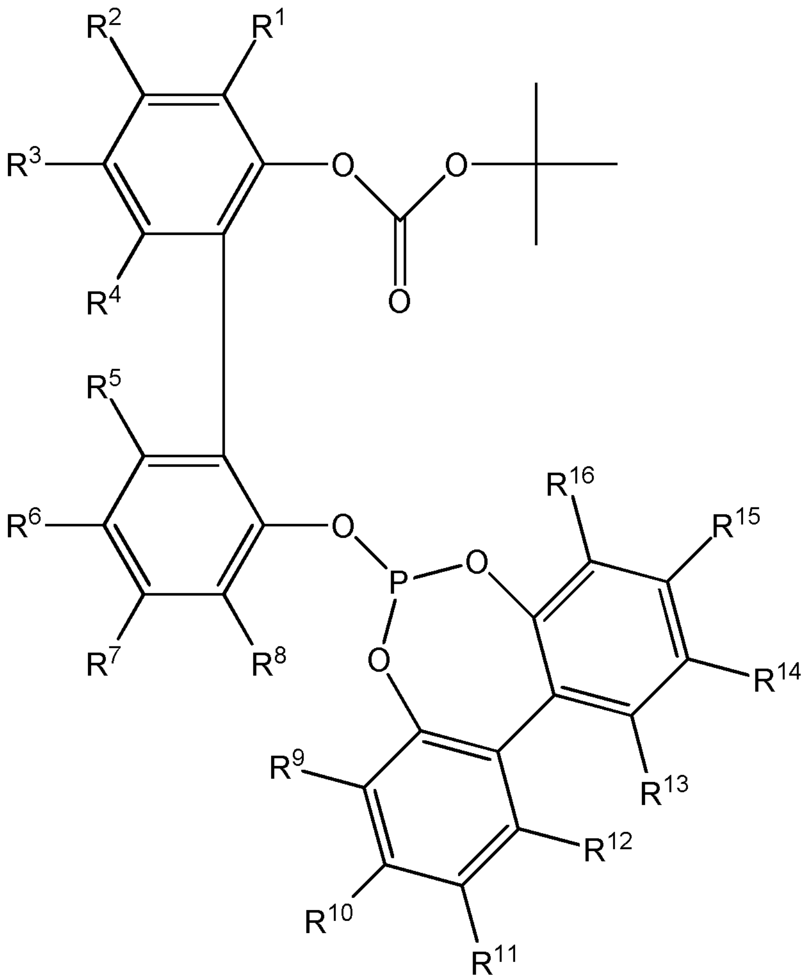

**Ie**

wobei $R^9$, $R^{10}$, $R^{11}$, $R^{12}$, $R^{13}$, $R^{14}$, $R^{15}$, $R^{16}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$ -$SO_3Na$ -$NO_2$, -CN, -$NH_2$, -N[($C_1$-$C_{12}$)-Alkyl]$_2$.

**9.** Gemisch nach einem der Ansprüche 1 bis 8, umfassend eine Verbindung gemäß der Struktur **If:**

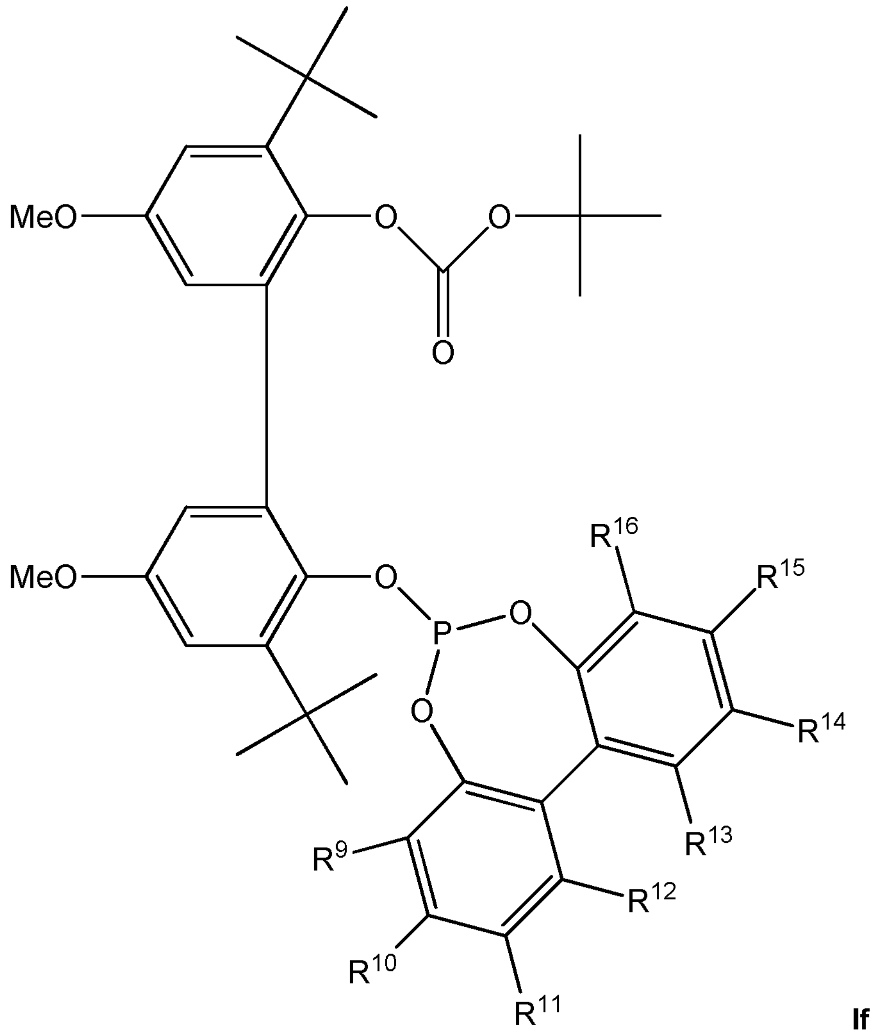

**If**

wobei $R^9$, $R^{10}$, $R^{11}$, $R^{12}$, $R^{13}$, $R^{14}$, $R^{15}$, $R^{16}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$ -$SO_3Na$ -$NO_2$, -CN, -$NH_2$, -N[($C_1$-$C_{12}$)-Alkyl]$_2$.

**10.** Gemisch nach einem der Ansprüche 1 bis 9,
wobei $R^{20}$, $R^{30}$, $R^{40}$ jeweils unabhängig voneinander ausgewählt sind aus: -($C_1$-$C_{12}$)-Alkyl, -($C_6$-$C_{20}$)-Aryl,
wobei die genannten Alkyl-, Arylgruppen substituiert sein können.

**11.** Gemisch nach einem der Ansprüche 1 bis 10,
umfassend eine Verbindung gemäß der Struktur **IIb:**

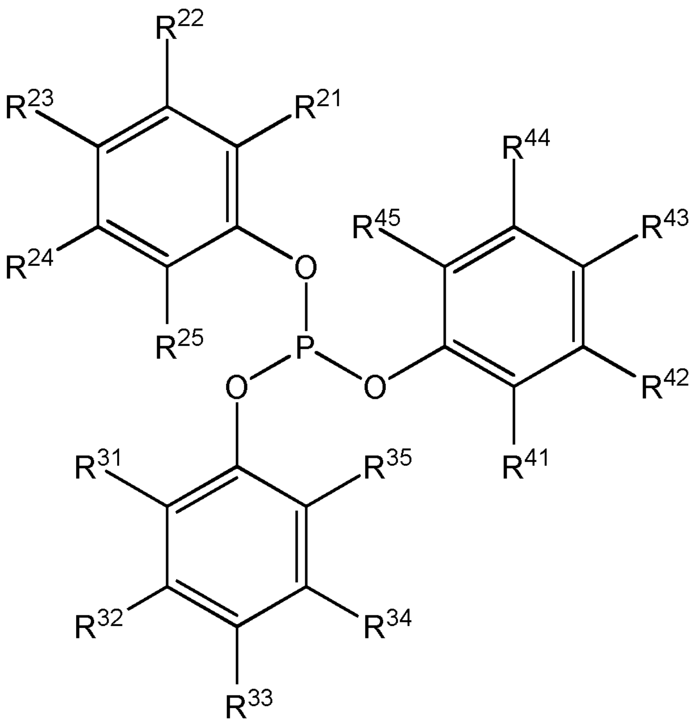

IIb

wobei
$R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl, -COOH, - OH, -$SO_3H$ -$SO_3Na$ -$NO_2$, -CN, -$NH_2$, -N[($C_1$-$C_{12}$)-Alkyl]$_2$.

**12.** Gemisch nach Anspruch 11,
wobei $R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

**13.** Komplexgemisch umfassend:

- ein Gemisch nach einem der Ansprüche 1 bis 12,
- ein Metallatom ausgewählt aus: Rh, Ru, Co, Ir.

**14.** Verwendung eines Gemisches nach einem der Ansprüche 1 bis 12, in einem Komplexgemisch zur Katalyse einer Hydroformylierungsreaktion.

**15.** Verfahren umfassend die Verfahrensschritte:

a) Vorlegen eines Olefins,
b) Zugabe eines Komplexgemisches nach Anspruch 13,
oder eines Gemisches nach einem der Ansprüche 1 bis 12 und einer Verbindung, welche ein Metallatom ausgewählt aus: Rh, Ru, Co, Ir aufweist,
c) Zuführen von $H_2$ und CO,
d) Erwärmen des Reaktionsgemisches, wobei das Olefin zu einem Aldehyd umgesetzt wird.

## Claims

**1.** Mixture comprising at least one of the two compounds of the structures **Ia** and **Ib:**

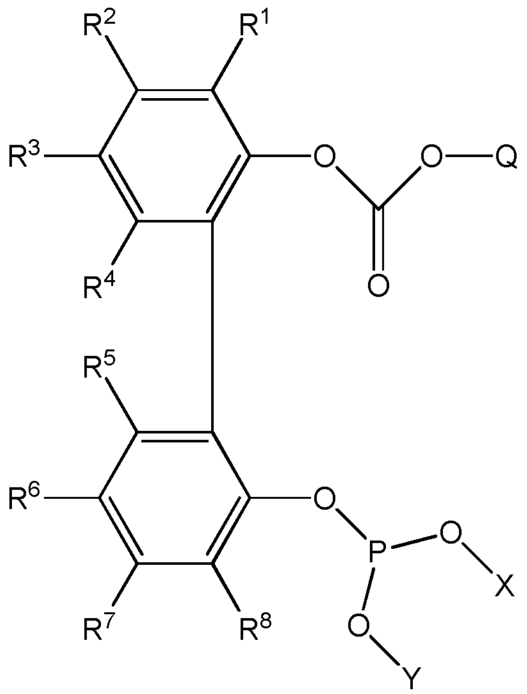

**Ia**

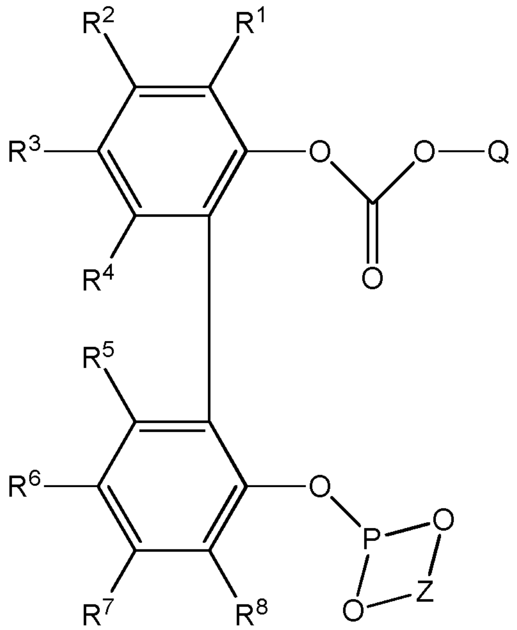

**Ib**

where

$R^1$, $R^2$, $R^3$, $R^4$, $R^5$, $R^6$, $R^7$, $R^8$ are each independently selected from: -H, -($C_1$-$C_{12}$)-alkyl, -O-($C_1$-$C_{12}$)-alkyl, -O-($C_6$-$C_{20}$) -aryl, -($C_6$-$C_{20}$)-aryl, halogen, COO-($C_1$-$C_{12}$)-alkyl, CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3$H, -$SO_3$Na, -$NO_2$, -CN, -$NH_2$, -N[($C_1$-$C_{12}$)-alkyl]$_2$;

X and Y are each independently selected from: -($C_1$-$C_{12}$)-alkyl, -($C_6$-$C_{20}$)-aryl, -($C_6$-$C_{20}$)-aryl-($C_1$-$C_{12}$)-alkyl, -($C_6$-$C_{20}$)-aryl-O-($C_1$-$C_{12}$)-alkyl, -($C_1$-$C_{12}$)-alkyl-($C_6$-$C_{20}$)-aryl, -($C_6$-$C_{20}$)-aryl-COO-($C_1$-$C_{12}$)-alkyl, -($C_6$-$C_{20}$)-aryl-CONH-($C_1$-$C_{12}$)-alkyl, -($C_6$-$C_{20}$)-aryl-CON[($C_1$-$C_{12}$)-alkyl]$_2$, -($C_4$-$C_{20}$)-heteroaryl, -($C_4$-$C_{20}$)-heteroar-

yl-$(C_1$-$C_{12})$-alkyl, -$(C_5$-$C_8)$-Cycloalkyl-$(C_4$-$C_{20})$-aryl-CO-$(C_6$-$C_{20})$-aryl,
Z is selected from: -$(C_1$-$C_{12})$-alkyl-, -$(C_6$-$C_{20})$-aryl-, -$(C_6$-$C_{20})$-aryl-$(C_1$-$C_{12})$-alkyl-, -$(C_1$-$C_{12})$-alkyl-$(C_6$-$C_{20})$-aryl-, - $(C_4$-$C_{20})$-heteroaryl-, -$(C_6$-$C_{20})$-aryl-CO-$(C_6$-$C_{20})$-aryl-, -$(C_6$-$C_{20})$-aryl- $(C_6$-$C_{20})$-aryl-;
Q is selected from: -$(C_1$-$C_{18})$-alkyl-, -$(C_1$-$C_{12})$-alkyl-$(C_1$-$C_{20})$-aryl-, -$(C_1$-$C_{18})$-haloalkyl-, -NH-$(C_1$-$C_{18})$-alkyl, where the alkyl, heteroalkyl, cycloalkyl, heterocycloalkyl, aryl and heteroaryl groups mentioned may be substituted,
and a compound of the structure **IIa:**

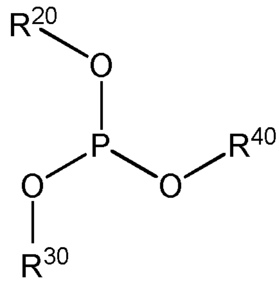

**IIa**

where
$R^{20}$, $R^{30}$, $R^{40}$ are each independently selected from: -$(C_1$-$C_{12})$-alkyl, -$(C_6$-$C_{20})$-aryl, -$(C_3$-$C_{12})$-Cycloalkyl, two $R^{20}$ and $R^{30}$ or $R^{20}$ and $R^{40}$ or $R^{30}$ and $R^{40}$ radicals may also be bridged to one another, and may have a -$(C_6$-$C_{20})$-aryl-$(C_6$-$C_{20})$-aryl unit,
where the alkyl, cycloalkyl and aryl groups mentioned may be substituted.

**2.** Mixture according to Claim 1,
where Q is selected from: - $(C_1$-$C_{12})$-alkyl-, -$(C_1$-$C_3)$-alkyl-$(C_1$-$C_3)$-aryl-, -$(C_1$-$C_{18})$-haloalkyl-, -NH-$(C_1$-$C_8)$-alkyl.

**3.** Mixture according to either of Claims 1 and 2, comprising at least one of the two compounds of one of the structures **Ic** and **Id:**

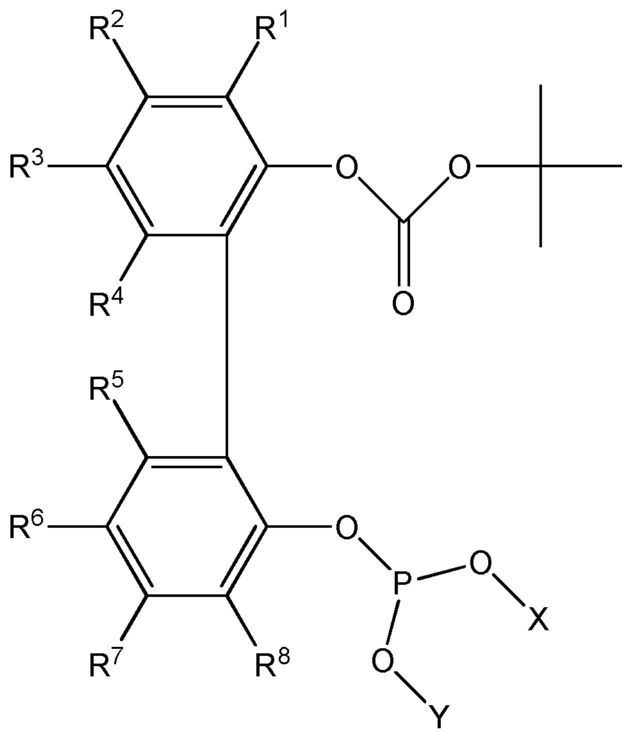

**Ic**

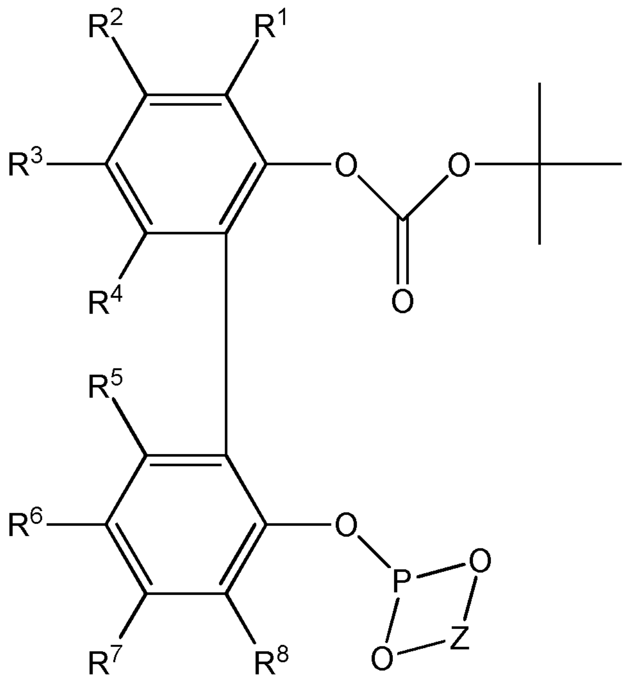

**Id**

4. Mixture according to any of Claims 1 to 3, where X and Y are each independently selected from: -($C_1$-$C_{12}$)-alkyl, -($C_6$-$C_{20}$)-aryl, -($C_6$-$C_{20}$)-aryl-($C_1$-$C_{12}$)-alkyl, - ($C_6$-$C_{20}$)-aryl-O-($C_1$-$C_{12}$)-alkyl, ($C_6$-$C_{20}$)-aryl-COO-($C_1$-$C_{12}$)-alkyl.

5. Mixture according to any of Claims 1 to 4, where Z is selected from: -($C_1$-$C_{12}$)-alkyl-, -($C_6$-$C_{20}$)-aryl-, -($C_6$-$C_{20}$)-aryl-($C_1$-$C_{12}$)-alkyl-, -($C_6$,-$C_{20}$)-aryl-CO-($C_6$-$C_{20}$)-aryl-, -($C_1$-$C_{12}$)-alkyl-($C_6$-$C_{20}$)-aryl-, -($C_6$-$C_{20}$)-aryl-($C_6$-$C_{20}$)-aryl-.

6. Mixture according to any of Claims 1 to 5, where $R^1$, $R^2$, $R^3$, $R^4$, $R^5$, $R^6$, $R^7$, $R^8$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl, -COO-($C_1$-$C_{12}$)-alkyl, -CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$NH_2$, -N[($C_1$-$C_{12}$)-alkyl]$_2$.

7. Mixture according to any of Claims 1 to 6, where X and Y are the same radicals.

8. Mixture according to any of Claims 1 to 7, comprising a compound of the structure **Ie:**

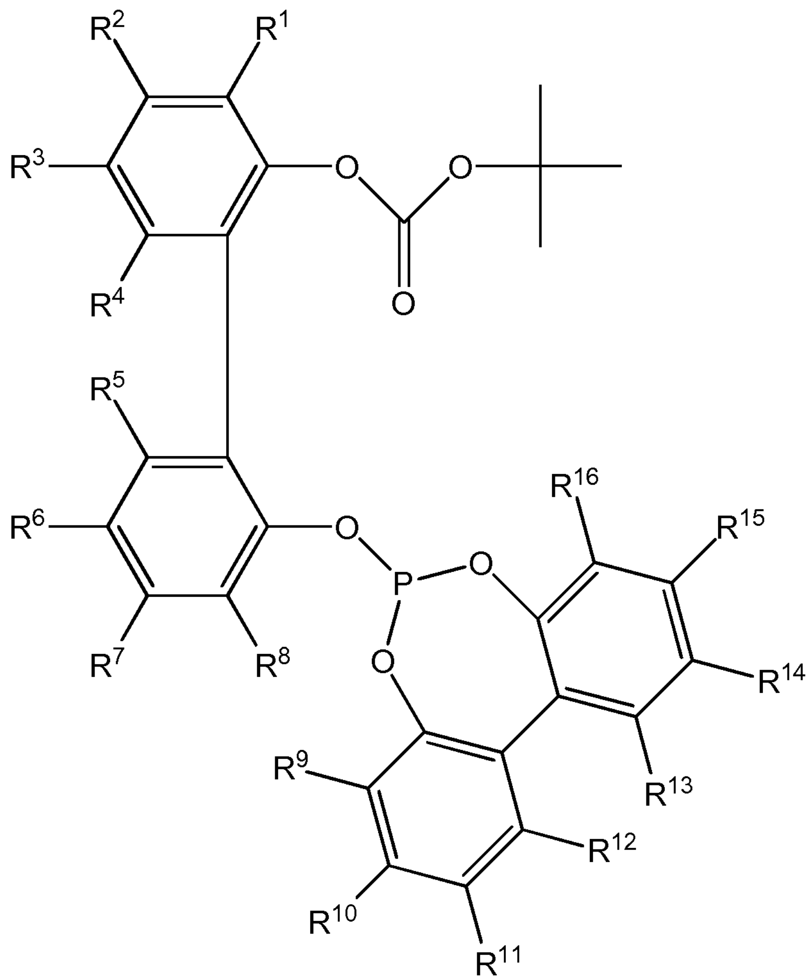

**Ie**

where $R^9$, $R^{10}$, $R^{11}$, $R^{12}$, $R^{13}$, $R^{14}$, $R^{15}$, $R^{16}$ are each independently selected from: -H, $-(C_1-C_{12})$-alkyl, $-O-(C_1-C_{12})$-alkyl, $-O-(C_6-C_{20})$-aryl, $-(C_6-C_{20})$-aryl, -halogen, $-COO-(C_1-C_{12})$-alkyl, $-CONH-(C_1-C_{12})$-alkyl, $-(C_6-C_{20})$-aryl-CON$[(C_1-C_{12})$-alkyl$]_2$, $-CO-(C_1-C_{12})$-alkyl, $-CO-(C_6-C_{20})$-aryl, -COOH, -OH, $-SO_3H$, $-SO_3Na$, $-NO_2$, -CN, $-NH_2$, $-N[(C_1-C_{12})$-alkyl$]_2$.

**9.** Mixture according to any of Claims 1 to 8, comprising a compound of the structure **If:**

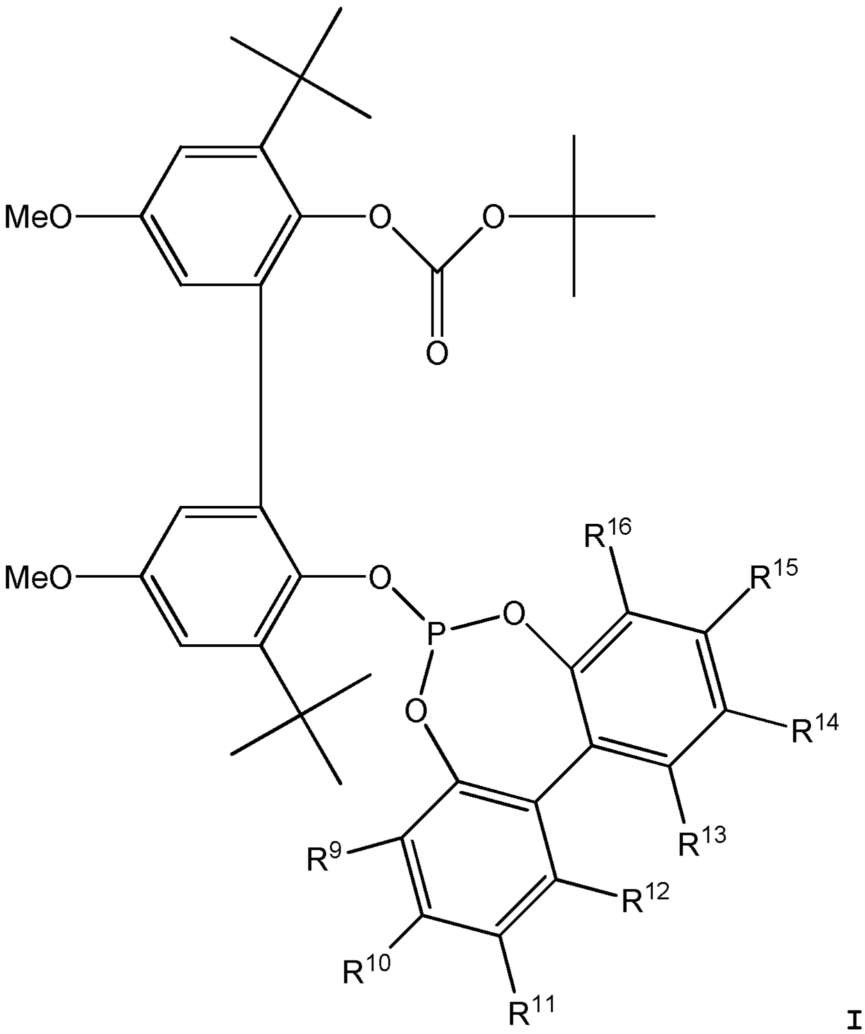

**If**

where $R^9$, $R^{10}$, $R^{11}$, $R^{12}$, $R^{13}$, $R^{14}$, $R^{15}$, $R^{16}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl, -halogen, -COO-($C_1$-$C_{12}$)-alkyl, -CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3H$, -$SO_3Na$, -$NO_2$, -CN, -$NH_2$, -N[($C_1$-$C_{12}$)-alkyl]$_2$.

**10.** Mixture according to any of Claims 1 to 9,

where $R^{20}$, $R^{30}$, $R^{40}$ are each independently selected from: -($C_1$-$C_{12}$)-alkyl,-($C_6$-$C_{20}$)-aryl, where the alkyl and aryl groups mentioned may be substituted.

**11.** Mixture according to any of Claims 1 to 10, comprising a compound of the structure **IIb**:

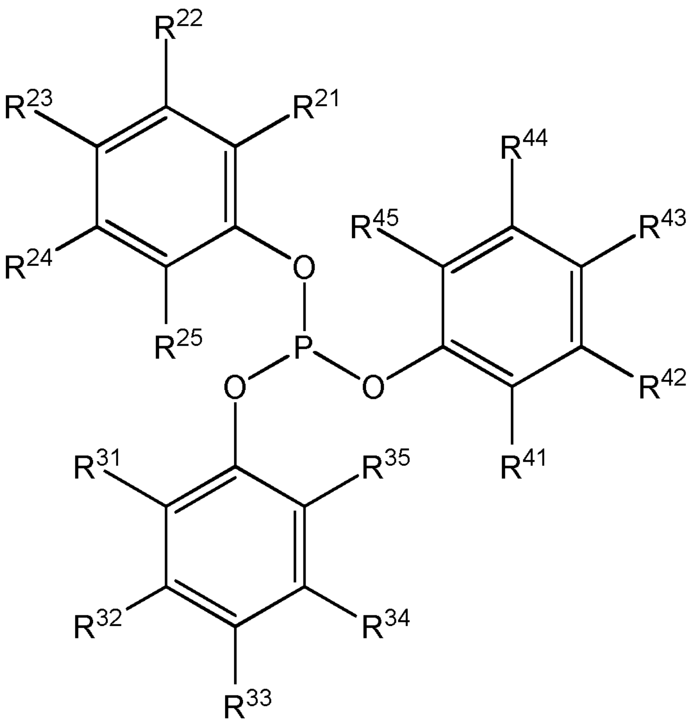

**IIb**

where
$R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ are each independently selected from: -H, -($C_1$-$C^{12}$)-alkyl, -O-($C_1$-$C_{12}$)-alkyl, -O-($C_6$-$C_{20}$)-aryl, -($C_6$-$C_{20}$)-aryl, -halogen, -COO-($C_1$-$C_{12}$)-alkyl, -CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3$H, -$SO_3$Na, -$NO_2$, -CN, -$NH_2$, -N[($C_1$-$C_{12}$)-alkyl]$_2$.

**12.** Mixture according to Claim 11,
where $R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ are each independently selected from: -H, -($C_1$-$C_{12}$)-alkyl, -O-($C_1$-$C_{12}$)-alkyl, -O-($C_6$-$C_{20}$)-aryl, -($C_6$-$C_{20}$)-aryl.

**13.** Complex mixture comprising:

- a mixture according to any of Claims 1 to 12,
- a metal atom selected from: Rh, Ru, Co, Ir.

**14.** Use of a mixture according to any of Claims 1 to 12 in a complex mixture for catalysis of a hydroformylation reaction.

**15.** Process comprising the process steps of:

a) initially charging an olefin,
b) adding a complex mixture according to Claim 13,
or a mixture according to any of Claims 1 to 12 and a compound including a metal atom selected from: Rh, Ru, Co, Ir,
c) feeding in $H_2$ and CO,
d) heating the reaction mixture, with conversion of the olefin to an aldehyde.

**Revendications**

**1.** Mélange comprenant au moins un des deux composés selon les structures Ia ou Ib :

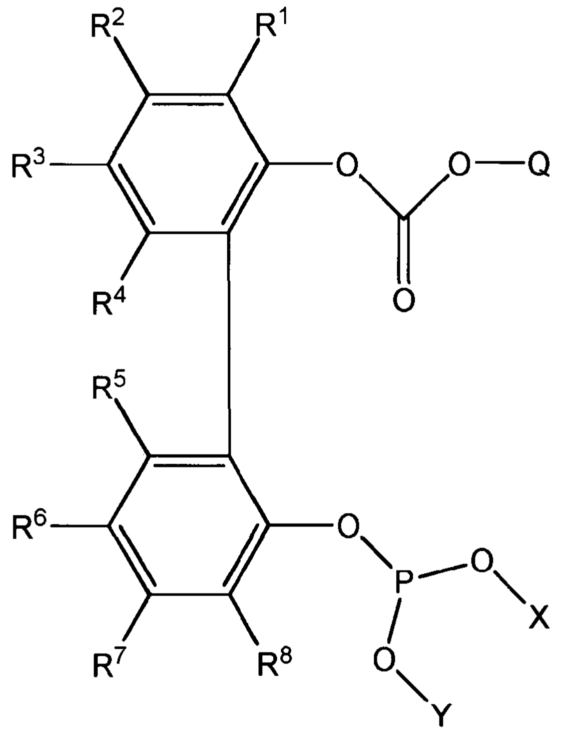

Ia

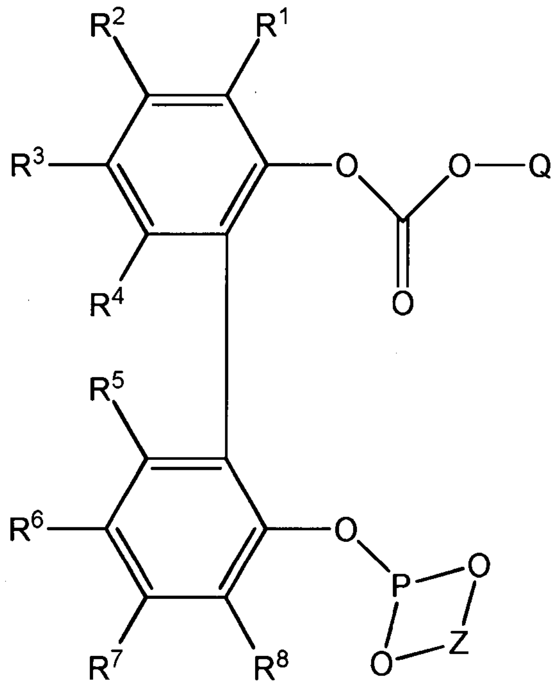

Ib

dans lesquelles

$R^1$, $R^2$, $R^3$, $R^4$, $R^5$, $R^6$, $R^7$, $R^8$ sont choisis, à chaque fois indépendamment les uns des autres, parmi : -H, -($C_1$-$C_{12}$)-alkyle, -O-($C_1$-$C_{12}$)-alkyle, -O-($C_6$-$C_{20}$)-aryle, - ($C_6$-$C_{20}$)-aryle, halogène, COO-($C_1$-$C_{12}$)-alkyle, CONH-($C_1$-$C_{12}$)-alkyle, -($C_6$-$C_{20}$)-aryl-CON[($C_1$-$C_{12}$)-alkyle]$_2$, -CO-($C_1$-$C_{12}$)-alkyle, -CO-($C_6$-$C_{20}$)-aryle,

-COOH, -OH, $-SO_3H$, $-SO_3Na$, $-NO_2$, -CN, $-NH_2$, - N[($C_1$-$C_{12}$)-alkyle]$_2$ ;
X et Y sont choisis, à chaque fois indépendamment l'un de l'autre, parmi : -($C_1$-$C_{12}$)-alkyle, -($C_6$-$C_{20}$)-aryle, -($C_6$-$C_{20}$)-aryl-($C_1$-$C_{12}$)-alkyle, -($C_6$-$C_{20}$)-aryl-O-($C_1$-$C_{12}$)-alkyle, -($C_1$-$C_{12}$)-alkyl-($C_6$-$C_{20}$)-aryle, - ($C_6$-$C_{20}$)-aryl-COO-($C_1$-$C_{12}$)-alkyle, -($C_6$-$C_{20}$)-aryl-CONH-($C_1$-$C_{12}$)-alkyle, -($C_6$-$C_{20}$)-aryl-CON[($C_1$-$C_{12}$)-alkyle]$_2$, -($C_4$-$C_{20}$)-hétéroaryle, -($C_4$-$C_{20}$)-hétéroaryl-($C_1$-$C_{12}$)-alkyle, -($C_5$-$C_8$)-cycloalkyl-($C_4$-$C_{20}$)-aryl-CO-($C_6$-$C_{20}$)-aryle,
Z est choisi parmi : -($C_1$-$C_{12}$)-alkyl-, -($C_6$-$C_{20}$)-aryl-, -($C_6$-$C_{20}$)-aryl-($C_1$-$C_{12}$)-alkyl-, -($C_1$-$C_{12}$)-alkyl-($C_6$-$C_{20}$)-aryl-, - ($C_4$-$C_{20}$)-hétéroaryl-, -($C_6$-$C_{20}$)-aryl-CO-($C_6$-$C_{20}$)-aryl-, -($C_6$-$C_{20}$)-aryl-($C_6$-$C_{20}$)-aryl-;
Q est choisi parmi : -($C_1$-$C_{18}$)-alkyle, -($C_1C_{12}$)-alkyl-($C_1$-$C_{20}$)-aryle, -($C_1$-$C_{18}$)-halogénoalkyle, -NH-($C_1$-$C_{18}$)-alkyle, les groupes alkyle, hétéroalkyle, cycloalkyle, hétérocycloalkyle, aryle et hétéroaryle mentionnés pouvant être substitués,
ainsi qu'un composé selon la structure IIa :

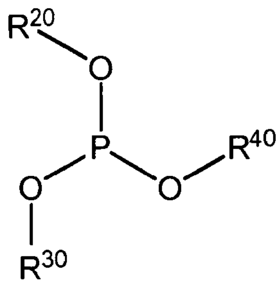

IIa

dans laquelle

$R^{20}$, $R^{30}$, $R^{40}$ sont choisis, à chaque fois indépendamment les uns des autres, parmi : - ($C_1$-$C_{12}$)-alkyle, -($C_6$-$C_{20}$)-aryle, -($C_3$-$C_{12}$)-cycloalkyle, en outre, deux radicaux $R^{20}$ et $R^{30}$ ou $R^{20}$ et $R^{40}$ ou $R^{30}$ et $R^{40}$ peuvent former un pont l'un avec l'autre et présenter une unité -($C_6$-$C_{20}$)-aryl-($C_6$-$C_{20}$)-aryle,
les groupes alkyle, cycloalkyle et aryle mentionnés pouvant être substitués.

2. Mélange selon la revendication 1, Q étant choisi parmi : -($C_1$-$C_{12}$)-alkyle, -($C_1$-$C_3$)-alkyl-($C_1$-$C_6$)-aryle, -($C_1$-$C_{18}$)-halogénoalkyle, -NH-($C_1$-$C_8$)-alkyle.

3. Mélange selon l'une quelconque des revendications 1 ou 2, comprenant au moins un des deux composés selon les structures Ic ou Id :

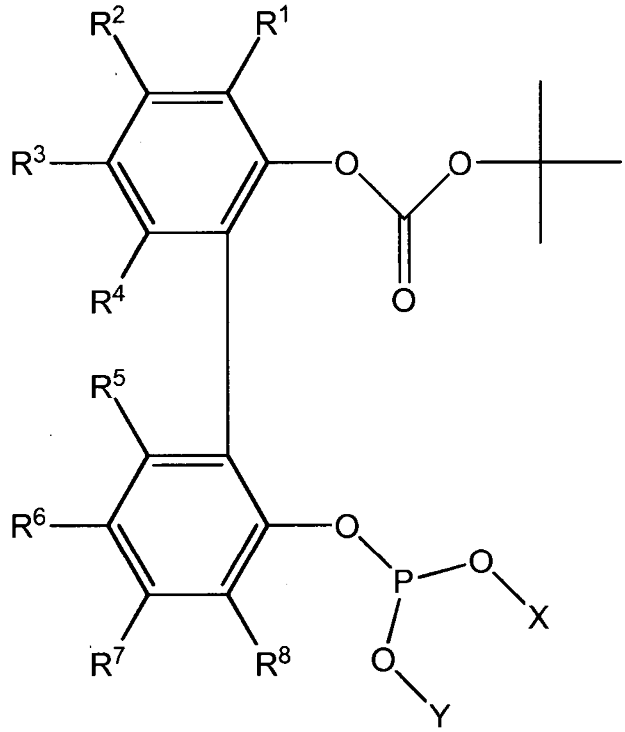

Ic

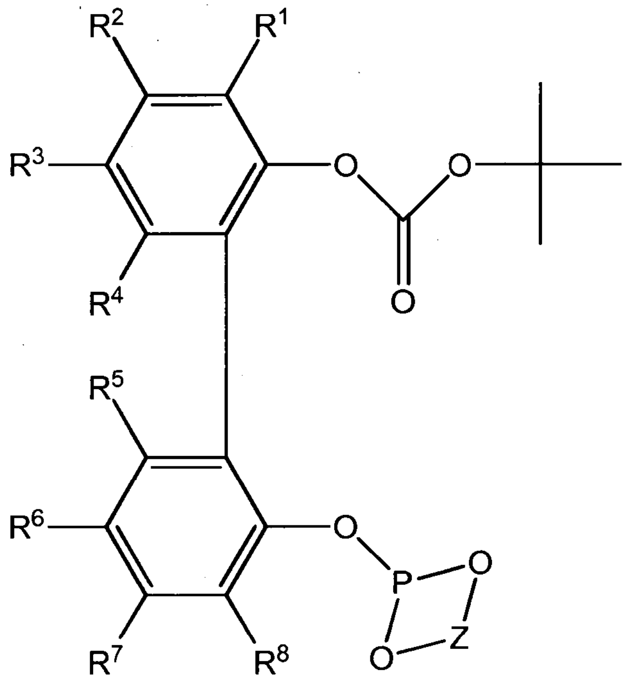

Id

**4.** Mélange selon l'une quelconque des revendications 1 à 3, X et Y étant choisis, à chaque fois indépendamment l'un de l'autre, parmi : -($C_1$-$C_{12}$)-alkyle,-($C_6$-$C_{20}$)-aryle,-($C_6$-$C_{20}$)-aryl-($C_1$-$C_{12}$)-alkyle, -($C_6$-$C_{20}$)-aryl-O-($C_1$-$C_{12}$)-alkyle, -($C_6$-$C_{20}$)-aryl-COO-($C_1$-$C_{12}$)-alkyle.

**5.** Mélange selon l'une quelconque des revendications 1 à 4, Z étant choisi parmi : -($C_1$-$C_{12}$)-alkyl-, -($C_6$-$C_{20}$)-aryl-, -($C_6$-$C_{20}$)-aryl-($C_1$-$C_{12}$)-alkyl-, -($C_6$-$C_{20}$)-aryl-CO-($C_6$-$C_{20}$)-aryl-, -($C_1$-$C_{12}$)-alkyl-($C_6$-$C_{20}$)-aryl-,

$-(C_6-C_{20})$-aryl-$(C_6-C_{20})$-aryl-.

6. Mélange selon l'une quelconque des revendications 1 à 5, $R^1$, $R^2$, $R^3$, $R^4$, $R^5$, $R^6$, $R^7$, $R^8$ étant choisis, à chaque fois indépendamment les uns des autres, parmi : -H, $-(C_1-C_{12})$-alkyle, $-O-(C_1-C_{12})$-alkyle, $-O-(C_6-C_{20})$-aryle, $-(C_6-C_{20})$-aryle, $-COO-(C_1-C_{12})$-alkyle, $-CONH-(C_1-C_{12})$-alkyle, $-(C_6-C_{20})$-aryl-$CON[(C_1-C_{12})$-alkyle$]_2$,$-CO-(C_1-C_{12})$-alkyle, $-CO-(C_6-C_{20})$-aryle, -COOH, -OH, $-NH_2$, $-N[(C_1-C_{12})$-alkyle$]_2$.

7. Mélange selon l'une quelconque des revendications 1 à 6, X et Y représentant les mêmes radicaux.

8. Mélange selon l'une quelconque des revendications 1 à 7, comprenant un composé selon la structure Ie :

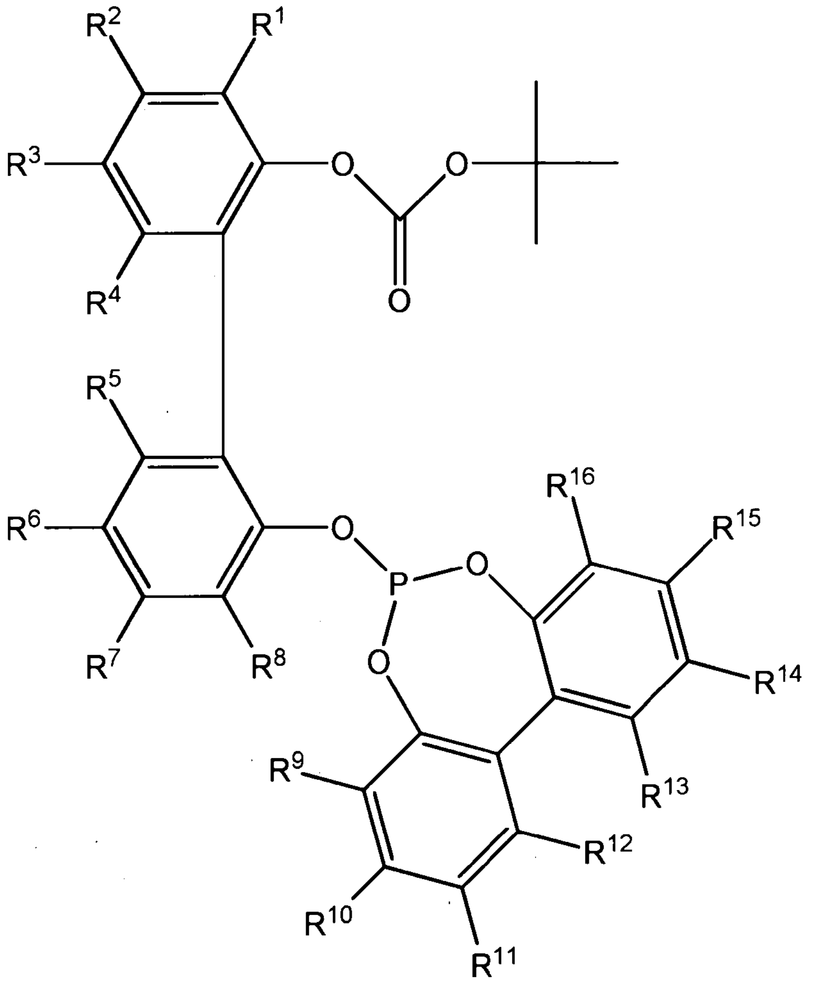

**Ie**

$R^9$, $R^{10}$, $R^{11}$, $R^{12}$, $R^{13}$, $R^{14}$, $R^{15}$, $R^{16}$ étant choisis, à chaque fois indépendamment les uns des autres, parmi : -H, $-(C_1-C_{12})$-alkyle, $-O-(C_1-C_{12})$-alkyle, $-O-(C_6-C_{20})$-aryle, $-(C_6-C_{20})$-aryle, -halogène, $-COO-(C_1-C_{12})$-alkyle, $-CONH-(C_1-C_{12})$-alkyle, $-(C_6-C_{20})$-aryl-$CON[(C_1-C_{12})$-alkyle$]_2$, $-CO-(C_1-C_{12})$-alkyle, $-CO-(C_6-C_{20})$-aryle, -COOH, -OH, $-SO_3H$, $-SO_3Na$, $-NO_2$, -CN, $-NH_2$, $-N[(C_1-C_{12})$-alkyle$]_2$.

9. Mélange selon l'une quelconque des revendications 1 à 8, comprenant un composé selon la structure If :

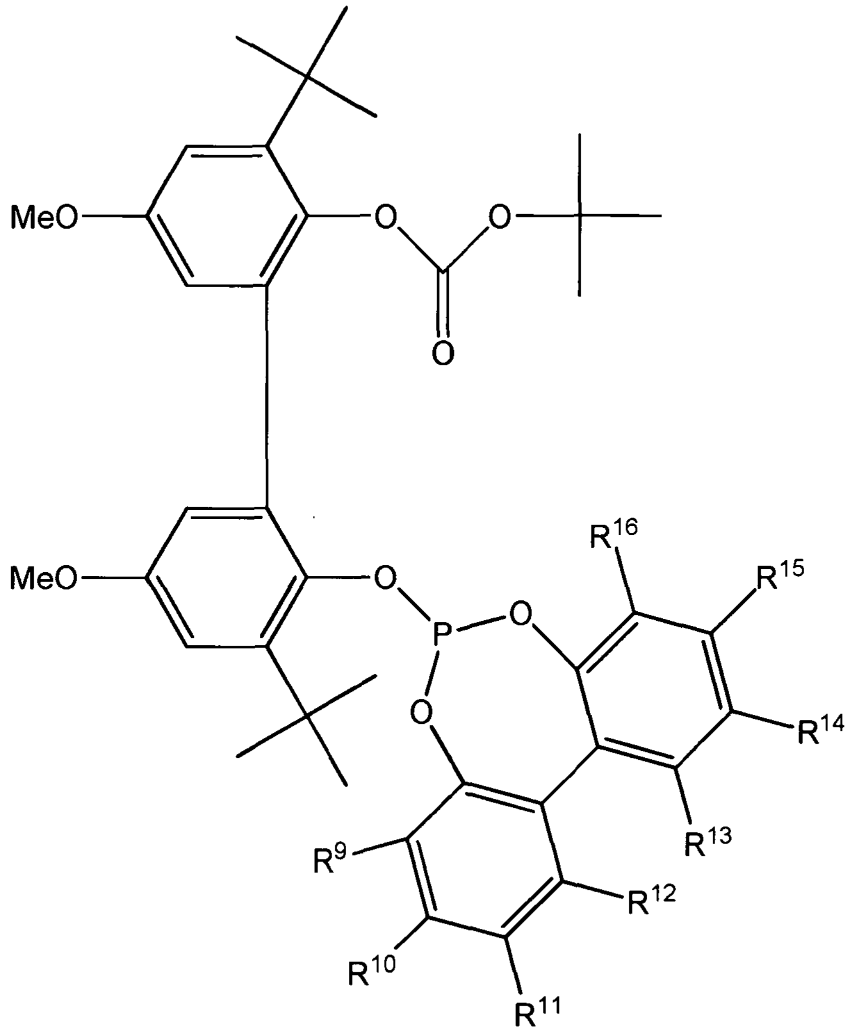

If

$R^9$, $R^{10}$, $R^{11}$, $R^{12}$, $R^{13}$, $R^{14}$, $R^{15}$, $R^{16}$ étant choisis, à chaque fois indépendamment les uns des autres, parmi : -H, $-(C_1-C_{12})$-alkyle, $-O-(C_1-C_{12})$-alkyle, $-O-(C_6-C_{20})$-aryle, $-(C_6-C_{20})$-aryle, -halogène, $-COO-(C_1-C_{12})$-alkyle, $-CONH-(C_1-C_{12})$-alkyle, $-(C_6-C_{20})$-aryl-$CON[(C_1-C_{12})$-alkyle$]_2$, $-CO-(C_1-C_{12})$-alkyle, $-CO-(C_6-C_{20})$-aryle, -COOH, -OH, $-SO_3H$, $-SO_3Na$, $-NO_2$, -CN, $-NH_2$, $-N[(C_1-C_{12})$-alkyle$]_2$.

**10.** Mélange selon l'une quelconque des revendications 1 à 9, $R^{20}$, $R^{30}$, $R^{40}$ étant choisis, à chaque fois indépendamment les uns des autres, parmi : $-(C_1-C_{12})$-alkyle, $-(C_6-C_{20})$-aryle, les groupes alkyle et aryle mentionnés pouvant être substitués.

**11.** Mélange selon l'une quelconque des revendications 1 à 10, comprenant un composé selon la structure IIb :

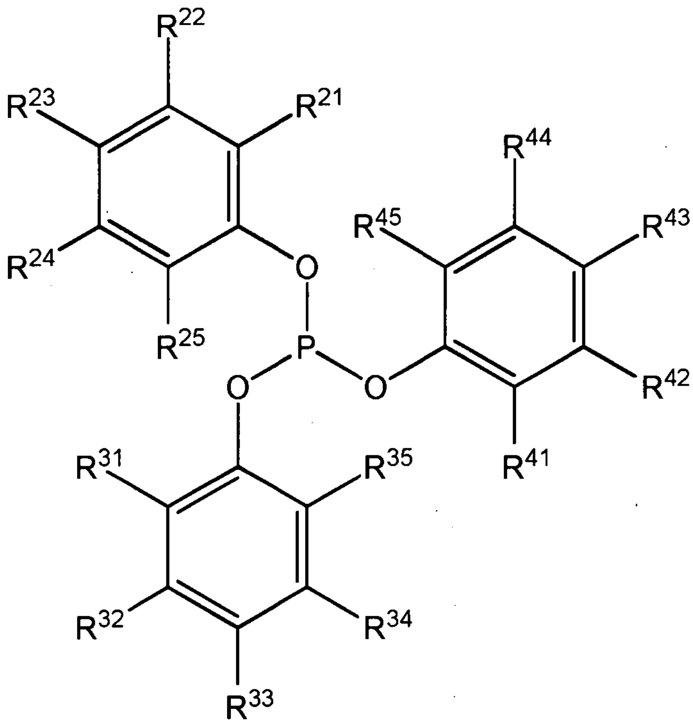

**IIb**

dans laquelle $R^{21}$, $R^{22}$, $R^{23}$, $R^{24}$, $R^{25}$, $R^{31}$, $R^{32}$, $R^{33}$, $R^{34}$, $R^{35}$, $R^{41}$ $R^{42}$, $R^{43}$, $R^{44}$, $R^{45}$ étant choisis, à chaque fois indépendamment les uns des autres, parmi : -H, -($C_1$-$C_{12}$)-alkyle, -O-($C_1$-$C_{12}$)-alkyle, -O-($C_6$-$C_{20}$)-aryle, -($C_6$-$C_{20}$)-aryle, -halogène, -COO-($C_1$-$C_{12}$)-alkyle, -CONH-($C_1$-$C_{12}$)-alkyle, -($C_6$-$C_{20}$)-aryl-CON[($C_1$-$C_{12}$)-alkyle]$_2$, -CO-($C_1$-$C_{12}$)-alkyle, -CO-($C_6$-$C_{20}$)-aryle, -COOH, -OH, -$SO_3$H, -$SO_3$Na, -$NO_2$, -CN, -$NH_2$, -N[($C_1$-$C_{12}$)-alkyle]$_2$.

**12.** Mélange selon la revendication 11, $R^{21}$, $R^{22}$, $R^{23}$, $R^{24}$, $R^{25}$, $R^{31}$, $R^{32}$, $R^{33}$, $R^{34}$, $R^{35}$, $R^{41}$, $R^{42}$, $R^{43}$, $R^{44}$, $R^{45}$ étant choisis, à chaque fois indépendamment les uns des autres, parmi : -H, -($C_1$-$C_{12}$)-alkyle, -O-($C_1$-$C_{12}$)-alkyle, -O-($C_6$-$C_{20}$)-aryle, -($C_6$-$C_{20}$)-aryle.

**13.** Mélange complexe, comprenant :

- un mélange selon l'une quelconque des revendications 1 à 12,
- un atome métallique choisi parmi : Rh, Ru, Co, Ir.

**14.** Utilisation d'un mélange selon l'une quelconque des revendications 1 à 12 dans un mélange complexe pour la catalyse d'une réaction d'hydroformylation.

**15.** Procédé comprenant les étapes de procédé :

a) disposition préalable d'une oléfine,
b) addition d'un mélange complexe selon la revendication 13 ou d'un mélange selon l'une quelconque des revendications 1 à 12 et d'un composé qui présente un atome métallique choisi parmi : Rh, Ru, Co, Ir,
c) introduction de $H_2$ et de CO,
d) chauffage du mélange réactionnel, l'oléfine étant transformée en aldéhyde.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20120253080 A
- EP 1099678 A


### In der Beschreibung aufgeführte Nicht-Patentliteratur


- **B. CORNILS ; W. A. HERRMANN.** Applied Homogeneous Catalysis with Organometallic Compounds. VCH, Weinheim, 1996, vol. 1 & 2
- **R. FRANKE ; D. SELENT ; A. BÖRNER.** Applied Hydroformylation. *Chem. Rev.,* 2012
- **B. L. HAYMORE ; A. VAN HASSELT ; R. BECK.** *Annals of the New York Acad. Sci.,* 1983, vol. 415, 159-175
- **P. W. N. M. VAN LEEUWEN.** Rhodium Catalyzed Hydroformylation. Kluwer, 2000
- *Klaus-Diether Wiese, Dietmar Obst, Top. Organomet. Chem.,* 2006, vol. 18, 1-33
- *Angew. Chem. Int. Ed.,* 2000, vol. 39 (9), 1639-1641
- **VAN LEEUWEN.** *Journal of Catalysis,* 2013, vol. 298, 198-205
- **W. L. F. ARMAREGO ; CHRISTINA CHAI ; BUTTERWORTH HEINEMANN.** Purification of Laboratory Chemicals. Elsevier, 2009
- **ROBIN K. HARRIS ; EDWIN D. BECKER ; SONIA M. CABRAL DE MENEZES ; ROBIN GOODFELLOW ; PIERRE GRANGER.** *Pure Appl. Chem.,* 2001, vol. 73, 1795-1818
- **ROBIN K. HARRIS ; EDWIN D. BECKER ; SONIA M. CABRAL DE MENEZES ; PIERRE GRANGER ; ROY E. HOFFMAN ; KURT W. ZILM.** *Pure Appl. Chem.,* 2008, vol. 80, 59-84